# EUROPEAN PATENT APPLICATION

(11) **EP 4 104 823 A1**
(43) Date of publication of application: **21.12.2022**
(21) Application number: 21753772.9
(22) Date of filing: 15.02.2021
(51) Int. Cl.: A61K 31/122, A23L 33/10, A61K 31/215, A61K 31/7004, A61P 35/00, A61P 35/02, A61P 35/04, A61P 43/00

(54) **INHIBITOR OF ASPARTIC ACID SYNTHESIS IN TUMOR CELLS, INHIBITOR OF SPHEROID FORMATION OF TUMOR CELLS, INHIBITOR OF TUMOR CELL METASTASIS, ACTIVITY ENHANCER OF GLYCOLYTIC INHIBITOR, AND PHARMACEUTICAL COMPOSITION FOR SUPPRESSING AND/OR PREVENTING TUMOR METASTASIS**

(30) Priority: 13.02.2020 JP 2020022827
(71) Applicant: CCI Holdings Inc., Seki-shi, Gifu 501-3923 (JP); Akao, Yukihiro, Gifu-shi, Gifu 500-8483 (JP)
(72) Inventor: HEISHIMA, Kazuki, Gifu-shi, Gifu 501-1131 (JP); ITO, Ryo, Seki-shi, Gifu 501-3923 (JP); HONDA, Kenji, Seki-shi, Gifu 501-3923 (JP); AKAO, Yukihiro, Gifu-shi, Gifu 500-8483 (JP)
(74) Representative: Hasegawa, Kan
(86) International application number: PCT/JP2021/005474
(87) International publication number: WO 2021/162126

(57) **Abstract**

Provided is a highly safe metastasis inhibitor of tumor cells that can be substituted for a conventionally known metastasis inhibitor of tumor cells.

A compound represented by Chemical Formula 1A or Chemical Formula 1B described in the description is used as an aspartic acid synthesis inhibitor in tumor cells, a spheroid formation inhibitor of tumor cells, or an action enhancer of glycolysis inhibitors. These inhibitors have a function as a highly potent metastasis inhibitor of tumor cells.

## Description

### TECHNICAL FIELD

The present invention relates to an inhibitor of aspartic acid synthesis in tumor cells, a spheroid formation inhibitor of tumor cells, a metastasis inhibitor of tumor cells, an action enhancer of glycolysis inhibitors, and a pharmaceutical composition for suppression and/or prevention of metastasis of a tumor.

### BACKGROUND ART

Currently, cancer is a top cause of death, and there is a trend that the number of cancer patients is increasing year by year. To deal with cancers, since an extract that is derived from a natural product having a proliferation inhibitory effect on tumor cells has been found and the safety is high as compared with conventional anticancer agents, it is expected to provide a cell growth inhibitory agent having few side effects, an antitumor agent containing these components (also referred to as an anticancer agent or a carcinostatic agent), and furthermore, a health food for preventing cancer.

In addition, there has been a trend that pets such as dogs are also regarded as family members in recent years, and in a survey in 2001, 64% of respondents in total consider that "pets are also family members", making their lifestyle close to that of humans. On the other hand, with an increase in average life span of pets, incidence of neoplastic diseases on them are also increasing. Particularly in dogs and cats, death rate due to cancer at an old age is high, and in some dog species and cat species, incidence of cancer may be higher than that in humans. Similar to the treatment of cancer on humans, treatment of cancer on pets includes surgery, chemotherapy, drug therapy, radiotherapy, immunotherapy, and the like. However, similar to humans, even when a pet suffers from cancer, symptoms are less likely to appear at an early stage, and symptoms such as loss of appetite and swelling of lymphoma often appear only after the cancer has progressed considerably. In addition, since the possibility of cancer metastasis cannot be eliminated even after the cancer is removed by surgery or the like, it is necessary to administer anticancer agents and perform periodic inspections. However, since a pet cannot recognize the importance of the anticancer agents or periodic inspections like a human, the administration of anticancer agents and the periodic inspections itself become a considerable burden for the pet and cause stress for the pet owner, too.

Therefore, there is a demand for a drug capable of suppressing metastasis of tumor cells after cancer treatment. For example, Patent Literature 1 proposes a metastasis inhibitor of tumor cells that contains a short-chain duplex oligonucleotide having an RNA interference action on MAST205 (serine threonine kinase) as an effective component.

Meanwhile, it has been conventionally known that petasin and S-petasin which are alicyclic compounds have an anticancer effect on malignant melanoma (Patent Literature 2). According to Patent Literature 2, it is presumed that these compounds express the above anticancer effect by suppressing MITF, which is a master transcription factor of enzymes and proteins necessary for melanin pigment generation, to suppress melanin production.

In addition, it has also been conventionally known that petasin, S-petasin, neopetasin, and neo-S-petasin, which are alicyclic compounds as well, have an inhibitory effect on proliferation of gastric cancer, colorectal cancer, and leukemia (Patent Literature 3). According to Patent Literature 3, it is presumed that these compounds express the above inhibitory effect on proliferation by inducing apoptosis in various tumor cells described above.

Furthermore, Non-Patent Literature 1 describes that the Petasites japonicus extract reduced viable cells into 23 to 47% in various cancer cell lines (K562, T24, A2058, and DLD-1), that petasin, neopetasin, neo-S-petasin, and S-petasin were identified as active components, that these components had similar cytostatic ability, and that the Petasites japonicus extract was orally administered to three cases of canine melanoma and one case showed regression of metastatic lung lesions. However, the above disclosure in Non-Patent Literature 1 does not mean that the Petasites japonicus extract functioned as a "metastasis inhibitor for suppressing melanoma lung metastasis". In other words, the above disclosure of Non-Patent Literature 1 merely indicates that the Petasites japonicus extract "suppressed proliferation of cancer cells in lung cancer lesion (caused by metastasis from melanoma lesion which is a primary focus)". Therefore, from the above disclosure of Non-Patent Literature 1, it is not possible to determine "whether or not the Petasites japonicus extract suppresses metastasis from the melanoma lesion which is the primary focus to lung".

In addition, Non-Patent Literature 2 discloses that petasin inhibited proliferation of colon cancer cell lines and suppressed migration and invasion thereof.

### Citation List

### Patent Literatures

Patent Literature 1: JP 2008-308407 A
Patent Literature 2: JP 2014-198683 A
Patent Literature 3: JP 2018-168119 A

### Non-Patent Literatures

Non-Patent Literature 1: Kazuki Hirashima et al., Antineoplastic activity of Petasites japonicus extract, Abstract Collection of The 77th Annual Meeting of the Japanese Cancer Association, 2018, Vol. 77, p. 692 [P-1125]
Non-Patent Literature 2: LYU, X. et al., Inhibitory effects of petasin on human colon carcinoma cells mediated by inactivation of Akt/mTOR pathway, Chinese medical journal, 2019, Vol. 132, No. 9, pp. 1071-1078

### SUMMARY OF INVENTION

### Technical Problem

The metastasis inhibitor of tumor cells described in Patent Literature 1 is a nucleic acid molecule. However, in recent years, nucleic acid molecules have not come to be generally used due to worries about safety thereof, and rather, labeling such as "non-use of recombinant" has been made on foods and there is a trend of refraining the use thereof.

The present invention has been made in view of the above circumstances, and an object of the present invention is to provide a highly safe metastasis inhibitor of tumor cells that can replace a conventionally known metastasis inhibitor of tumor cells.

### Solution to Problem

The present inventors have intensively studied to solve the above problems. In the process, when various compounds were searched for the metastasis inhibitory effect on tumor cells, it was surprisingly found that neopetasin, which is a compound disclosed in Patent Literature 3, exhibits a very potent metastasis inhibitory function on tumor cells. Furthermore, as a result of further studying the mechanism by which neopetasin exhibits such a potent metastasis inhibitory function, it was found that neopetasin exhibits the metastasis inhibitory function described above by strongly inhibiting the aspartic acid synthesis in tumor cells and the spheroid formation by tumor cells. In addition, based on these findings, the present inventors have completed the present invention.

According to an aspect of the present invention, there is provided an aspartic acid synthesis inhibitor in tumor cells that contains a compound (preferably petasin or neopetasin) represented by the following Chemical Formula 1A or the following Chemical Formula 1B as an effective component (the first aspect): 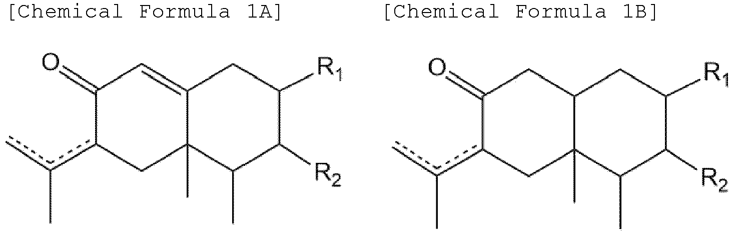 wherein R₁ and R₂ are each independently a hydrogen atom, a hydroxyl group or an optionally substituted hydrocarbon group. Furthermore, one of the two adjacent chemical bonds indicated by dotted lines is a single bond, and the other one is a double bond.

According to another aspect of the present invention, there is provided a spheroid formation inhibitor of tumor cells that contains the compound described above (preferably petasin or neopetasin) as an effective component (the second aspect).

According to still another aspect of the present invention, there is provided a metastasis inhibitor (for example, to lung) of tumor cells (for example, cells of malignant melanoma), that contain at least one of the agents described above.

According to still another aspect of the present invention, there is provided an action enhancer of glycolysis inhibitors that contains the compound described above (preferably petasin or neopetasin) as an effective component.

According to still another aspect of the present invention, there is provided a pharmaceutical composition for suppression and/or prevention of metastasis of a tumor that contains the agent according to any one of the aspects described above.

According to still another aspect of the present invention, there is provided a food or drink composition for suppressing metastasis of tumor cells, that contains the compound described above (preferably petasin or neopetasin) as an effective component.

### Advantageous Effects of the Invention

According to the present invention, there is provided a highly safe metastasis inhibitor of tumor cells that can replace a conventionally known metastasis inhibitor of tumor cells.

### BRIEF DESCRIPTION OF DRAWINGS

Fig. 1A is a graph showing results of evaluating the inhibitory effect on proliferation of petasin, S-petasin, neopetasin, and neo-S-petasin on mouse or canine-derived malignant melanoma cells in Test Example 1 of the Examples to be described later.
Fig. 1B is a graph showing results of evaluating the inhibitory effect on proliferation of neopetasin on mouse malignant melanoma cells in Test Example 1 of the Examples to be described later.
Fig. 1C is a graph showing results of comparing the action of neopetasin on normal human skin-derived fibroblasts with the action on tumor cells in Test Example 1 of the Examples to be described later.
Fig. 2A is a graph showing the results of examining the metastasis inhibitory effect of neopetasin using a mouse malignant melanoma lung metastasis model in Test Example 2 of the Examples to be described later, and a photograph of extracted lungs.
Fig. 2B is a photomicrograph that shows observation results of a section prepared from the tissue of each organ (heart, normal site of lung, cerebrum, kidney, liver, and spleen) when a model mouse to which neopetasin or corn oil had been administered was dissected in Test Example 2 of the Examples to be described later.
Fig. 3 is a graph showing results of examining an influence of neopetasin on anticancer action of 2-deoxy-D-glucose (2-DG) on tumor cells in Test Example 3 of the Examples to be described later.
Fig. 4 is a photomicrograph showing results of examining an influence of neopetasin on spheroid formation ability of B16F10 cells by a spheroid formation assay in Test Example 4 of the Examples to be described later.
Fig. 5 is a photomicrograph showing results of examining an influence of neopetasin on migration action of tumor cells by a scratch wound healing assay in Test Example 5 of the Examples to be described later.
Fig. 6 is a graph showing results of examining signaling pathways affected by neopetasin treatment by KEGG Pathway analysis using a microarray in Test Example 6 of the Examples to be described later.
Fig. 7 is a heat map showing a gene having a particularly large variation among the variable pathways shown in Fig. 6.
Fig. 8 is a heat map showing results of metabolomic analysis (amino acids) of B16F10 cells treated with neopetasin in Test Example 7 of the Examples to be described later.
Fig. 9A is a fluorescence micrograph showing results of evaluating an influence of neopetasin on mitochondria in B16F10 cells in Test Example 8 of the Examples to be described later.
Fig. 9B is a graph showing results of evaluating an influence of neopetasin on mitochondria in B16F10 cells by using an image cytometer in Test Example 8 of the Examples to be described later.
Fig. 9C is a photograph showing observation results of cells treated with each of neopetasin (3 µM) and metformin (5000 µM) by using an electron microscope in Test Example 8 of the Examples to be described later.
Fig. 10 is a graph showing results of examining how much relative activity of each of Complex I, Complex II, Complex II/III, Complex IV, and Complex V constituting the mitochondrial respiratory chain is reduced by neopetasin treatment in Test Example 9 of the Examples to be described later.
Fig. 11 is an electrophoretic photograph showing results of examining changes in expression of various cancer-related proteins in neopetasin-treated cells by Western blotting in Test Example 10 of the Examples to be described later.
Fig. 12 is an electrophoretic photograph showing results of examining temporal changes of expression of various cancer metabolism/proliferation/metastasis related proteins (AMPK, FAK, Akt, ERK1/2) in B16F10 cells treated with neopetasin or metformin by Western blotting in Test Example 11 of the Examples to be described later.
Fig. 13 is a view showing procedures of treatment in creating a mouse natural metastasis model that causes all processes of lung metastasis from a primary tumor in Test Example 12 of the Examples to be described later.
Fig. 14 is a macroscopic photograph of an extracted lung showing results of examining the metastasis inhibitory effect of neopetasin by using a mouse natural metastasis model in Test Example 12 of the Examples to be described later.
Fig. 15 is a graph showing results of examining the metastasis inhibitory effect of neopetasin by using a mouse natural metastasis model (counting results of lung metastasis lesions) in Test Example 12 of the Examples to be described later.
Fig. 16 is a photograph of an axillary lymph node extracted, showing results of examining the metastasis inhibitory effect of neopetasin by using a mouse natural metastasis model in Test Example 12 of the Examples to be described later.
Fig. 17 is a graph showing results of examining the metastasis inhibitory effect of neopetasin by using a mouse natural metastasis model (measurement results of weight of the axillary lymph node) in Test Example 12 of the Examples to be described later.
Fig. 18 is a graph showing results of examining an influence on body weight of a mouse when neopetasin is administered to a mouse natural metastasis model in Test Example 12 of the Examples to be described later.
Fig. 19 shows a protocol in Test Example 13 of the Examples to be described later, in which neopetasin (NP) was previously reacted with B16F10 cells derived from mouse melanoma at a concentration of 3 µM in vitro, then the number of living cells was uniformly aligned and injected into tail vein of a mouse, no treatment was performed thereafter, and the number of metastasis lesions formed in lung was counted after a 2-week non-treatment period.
Fig. 20 is a graph showing results of Test Example 13 of the Examples to be described later and observation photographs of extracted lungs.
Fig. 21 is a graph showing results of measuring IC₅₀ values in a cell proliferation inhibition assay for petasin, neopetasin, S-petasin, and S-neopetasin in addition to the Petasites japonicus extract (the whole extract) in Test Example 14 of the Examples to be described later.

### DESCRIPTION OF EMBODIMENTS

Hereinafter, embodiments of the present invention will be explained. Note that the present invention is not limited only to the following embodiments. In the present description, "X to Y" indicating a range includes X and Y, and means "X and more as well as Y and less". In addition, unless otherwise specified, operations and measurements of physical properties and the like are performed under conditions of room temperature (20 to 25°C)/relative humidity 40 to 50% RH.

The first aspect of the present invention is an aspartic acid synthesis inhibitor in tumor cells that contains a compound represented by the Chemical Formula 1A or Chemical Formula 1B described above as an effective component. Furthermore, the second aspect of the present invention is a spheroid formation inhibitor of tumor cells that contains a compound represented by the Chemical Formula 1A or Chemical Formula 1B described above as an effective component.

### [Compound Used as Effective Component]

Here, to start with, a compound represented by the following Chemical Formula 1A or Chemical Formula 1B (hereinafter, also referred to as a "compound of the present invention") used as an effective component will be explained. Furthermore, in the present invention, "containing a compound represented by Chemical Formula 1A or Chemical Formula 1B" is a concept including any of a form containing a compound represented by the Chemical Formula 1A, a form containing a compound represented by the Chemical Formula 1B, and a form containing both a compound represented by the Chemical Formula 1A and a compound represented by the Chemical Formula 1B. Moreover, the "hydrocarbon group optionally having a substituent" is a concept including a form in which a hydrocarbon is bonded to a cyclohexyl skeleton via a hetero atom (an oxygen atom, a nitrogen atom, a sulfur atom, and the like).

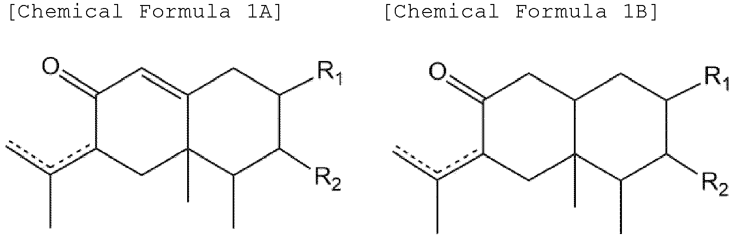

In the Chemical Formula 1A and Chemical Formula 1B, R₁ and R₂ are each independently a hydrogen atom, a hydroxyl group, or an optionally substituted hydrocarbon group. Furthermore, one of the two adjacent chemical bonds indicated by dotted lines is a single bond, and the other one is a double bond.

In the present description, the compound represented by the Chemical Formula 1A or Chemical Formula 1B is preferably a compound represented by the following Chemical Formula 2A or Chemical Formula 2B.

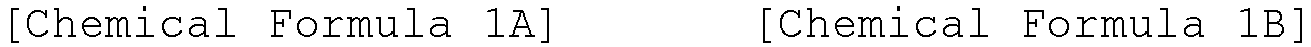

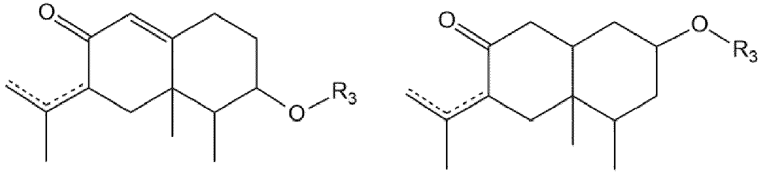

In the Chemical Formula 2A and Chemical Formula 2B, R₃ is a hydrogen atom or an optionally substituted hydrocarbon group. Furthermore, one of the two adjacent chemical bonds indicated by dotted lines is a single bond, and the other one is a double bond.

In addition, in the present description, the compound represented by the Chemical Formula 1A or Chemical Formula 1B is preferably a compound represented by the following Chemical Formula 3A.

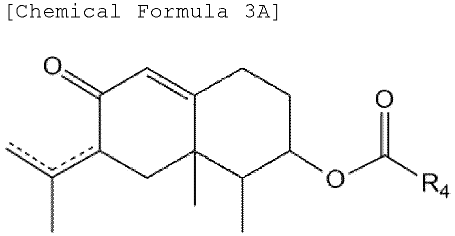

In the Chemical Formula 3A, R₄ is hydrogen or a hydrocarbon group optionally having a substituent. Furthermore, one of the two adjacent chemical bonds indicated by dotted lines is a single bond, and the other one is a double bond.

Furthermore, in the present description, from the viewpoint of various activity strengths, the compound represented by the Chemical Formula 1A or Chemical Formula 1B is preferably a compound represented by any of the following Chemical Formula 4A to Chemical Formula 6A, and more preferably a compound (petasin) represented by the Chemical Formula 4A or a compound (neopetasin) represented by the Chemical Formula 5A. Moreover, from the viewpoint of good activity strength, the compound (neopetasin) represented by the Chemical Formula 5A is most preferable.

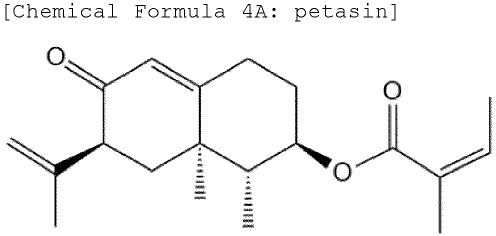

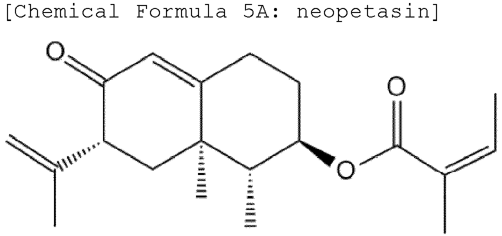

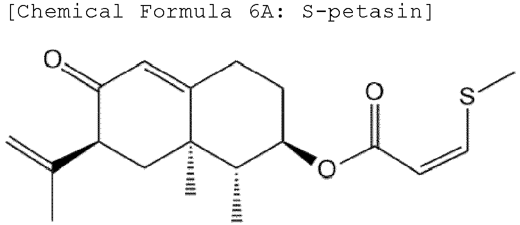

Note that the compound according to the present invention described above may be synthesized according to a conventional method or may be purified from such an extract as long as it can be extracted from a natural product in consideration of the common technical knowledge at the time of filing of the present application.

### [Application According to Each Aspect of the Present Invention]

Subsequently, an application according to each aspect of the present invention described above will be explained.

As described above, petasin and S-petasin are known to have the anticancer effect on malignant melanoma (Patent Literature 2), and moreover, petasin, S-petasin, neopetasin, and neo-S-petasin are known to have the inhibitory effect on proliferation of gastric cancer, colorectal cancer, and leukemia (Patent Literature 3). However, Patent Literature 2 and Patent Literature 3 do not disclose at all that such inhibitory effect on proliferation of tumor cells exhibited by the compound of the present invention is caused by action mechanisms such as inhibition of mitochondrial respiratory chain Complex I in tumor cells, inhibition of aspartic acid synthesis, and suppression of expression of various cancer-related molecules.

On the other hand, the present inventors have found for the first time that the compound according to the present invention exhibits various actions on tumor cells, and have completed the present invention.
- To start with, as a result of investigations by the present inventors, it was found in an in vivo experiment using the mouse malignant melanoma lung metastasis model that, the compound of the present invention exhibits a very potent metastasis inhibitory function on tumor cells. Then, by a lung metastasis test of tumor cells using B16F10 cells, which are highly metastatic malignant melanoma cell lines in mice, it was also confirmed that the compound of the present invention exhibited a very potent inhibitory effect on the migration of tumor cells (Test Example 2 to be described later). In other words, according to an aspect of the present invention, there is provided a metastasis inhibitor of tumor cells that contains at least one of the compounds according to the present invention described above as an effective component. Similarly, according to the present invention, there is also provided a method for inhibiting metastasis of tumor cells, that includes administering the compound according to the present invention described above (preferably, neopetasin) to a patient in need thereof. Furthermore, according to the present invention, the compound (preferably, neopetasin) according to the present invention described above for use in suppressing the metastasis of tumor cells is also provided. Moreover, according to the present invention, an application of the compound (preferably, neopetasin) according to the present invention described above to suppress metastasis of tumor cells is also provided.

In the present description, "migration of cells" or "cell migration" means that cells move from an original position to another position due to the mobility thereof. In other words, the migration of tumor cells is a phenomenon in which the tumor cells actively move. The migration of tumor cells is a phenomenon that also occurs in cancer metastasis. It is considered that the metastasis inhibitor of tumor cells according to one aspect of the present invention can suppress/inhibit a phenomenon (metastasis) in which the tumor cells migrate (move) from a place where the tumor cells are generated (a primary focus) and form a cancer or a tumor again at a remote site, by suppressing/inhibiting the migration of the tumor cells. Note that types of cell migration include chemotaxis in which cells migrate by a concentration gradient of a chemotactic factor such as a chemokine, haptotaxis in which cells migrate according to the concentration gradient of a chemotactic factor bound to a cell adhesion site or an extracellular matrix, wound healing in which cells migrate across wounds, and cell invasion in which cells migrate to adjacent tissues through extracellular matrix (ECM) via the ECM degradation or proteolysis. Therefore, migration inhibitory effect on tumor cells can be evaluated using a known method, for example, inhibition of membrane ruffle formation, a wound healing assay, or using a commercially available cell migration measurement system. In the present description, the metastasis inhibitory effect on tumor cells is evaluated by the migration inhibitory effect on tumor cells, and specifically evaluated using a wound healing assay (Test Example 5 of the examples to be described later).

The metastasis inhibitor of tumor cells according to the present invention can suppress and/or prevent metastasis of tumor cells by suppressing and inhibiting migration of tumor cells. In the present description, "metastasis of tumor cells" means that the tumor cells separate from the primary focus, move to another tissue or organ, and proliferate there. In addition, "suppression of metastasis of tumor cells" or "tumor cell metastasis suppression" means suppression of a phenomenon (metastasis) in which the tumor cells move (migrate) from a place where the tumor cells are generated (a primary focus) and form a tumor again at a remote site. Here, in general, the "anticancer agent" is administered to a patient having a cancer lesion to suppress proliferation of cancer cells in the cancer lesion. On the other hand, the "metastasis inhibitor of tumor cells" described in the present description is for suppressing metastasis of cancer cells from a cancer lesion to another tissue, and whether or not a certain agent suppresses proliferation of cancer cells is irrelevant to whether or not the agent is a "metastasis inhibitor". Then, while an administration target of the "anticancer agent" is a patient having a cancer lesion, it is sufficiently assumed that targets to be administered of the "metastasis inhibitor" is administered not only to a patient having a cancer lesion but also to a patient not having a cancer lesion (at least apparently) after receiving a surgery, a radiotherapy, or the like. This means that, for example, in an answer to "Q8: I hear that metastasized cancer is difficult to cure, but why?" on the "Cancer Metastasis Q&A" on the website of The Japanese Association for Metastasis Research, URL: http://jamr.umin.ac.jp/qa/a08.html (last accessed on February 12, 2021), it is apparent from this description that "Anticancer agents also give damage to normal tissues, so there is a problem that the dosage cannot be increased. On the other hand, since metastasis is a phenomenon that occurs only in cancer, it is expected that anticancer agents that block only metastasis with few side effects will be developed in the future. If such a metastasis inhibitor can be obtained, a dream treatment is expected such that the primary focus can be treated with surgery and the metastasis can be treated with a metastasis inhibitor." As described above, since it is sufficiently assumed that the "metastasis inhibitor of tumor cells" and the "anticancer agent" are for different targets to be administered, they can be distinguished in terms of "application to a specific disease for which usages such as administration timing were specified".

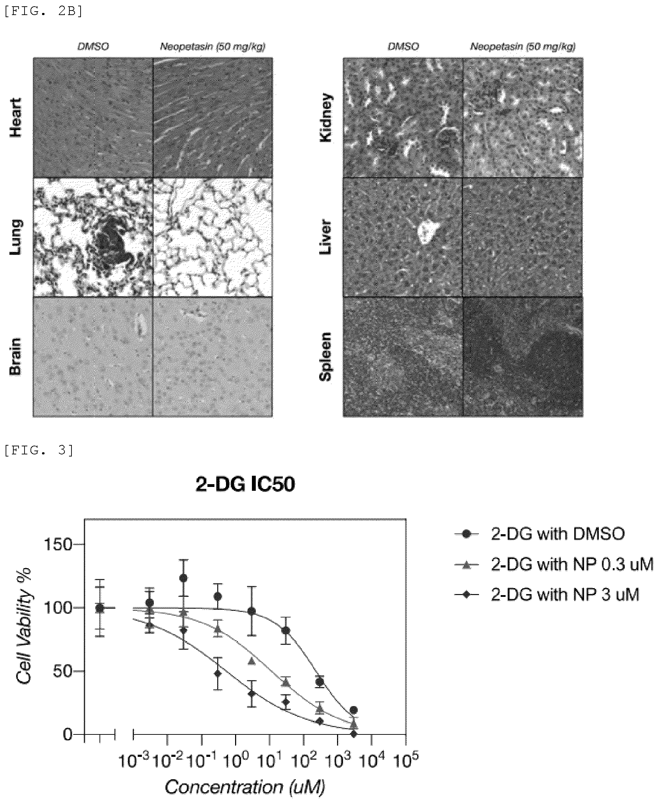

In addition, the proposition of "When proliferation of tumor cells is suppressed by anticancer agent, metastasis of tumor cells is also suppressed." is also incorrect. This is also apparent, for example, from the description of Paez-Ribes, M. et al. Antiangiogenic Therapy Elicits Malignant Progression of Tumors to Increased Local Invasion and Distant Metastasis.Cancer Cell 15, (2008). That is, the literature describes that, when mice were treated with Sunitinib, an anti-VEGFR2 inhibitor, proliferation of the primary tumor was suppressed, while metastasis of tumor cells to a lymph node and lung was promoted (Fig. 3 in the literature) . From this, it can be seen that the proposition of "When proliferation of tumor cells is suppressed by anticancer agents, metastasis of tumor cells is also suppressed." is not always correct, but may rather lead to opposite results.

Here, types of cancers targeted for metastasis inhibition of tumor cells is not particularly limited. Examples include cancers of nervous system (for example, cerebral tumor and cervical cancer); cancers of digestive system (for example, oral cavity cancer, pharyngeal cancer, esophageal cancer, gastric cancer, hepatic cancer, gallbladder cancer, biliary tract cancer, spleen cancer, colorectal cancer, small intestine cancer, duodenal cancer, colon cancer, colon adenocarcinoma, rectal cancer, pancreatic cancer, and liver cancer); cancers of musculoskeletal system (for example, sarcoma, osteosarcoma, and myeloma); cancers of urinary system (for example, bladder cancer and renal cancer); cancers of reproductive system (for example, breast cancer, uterine cancer, ovarian cancer, testicular cancer, and prostate cancer); cancers of respiratory system (for example, lung cancer); cancers of hematopoietic system (for example, acute or chronic myeloid leukemia, acute promyelocytic leukemia, leukemia such as acute or chronic lymphocytic leukemia, malignant lymphoma (lymphosarcoma), angiosarcoma, multiple myeloma, myelodysplastic syndrome, primary myelofibrosis, and hemangiopericytoma); thyroid cancer, parathyroid cancer, tongue cancer, malignant melanoma (melanoma), mastocytoma, skin histiocytoma, lipoma, follicular tumor, skin papilloma, sebaceous adenoma, basal cell cancer, and the like. Among them, targets of metastasis inhibition of tumor cells according to the present invention are preferably cancers of hematopoietic system, cancers of digestive system, cancer of reproductive system, malignant melanoma (melanoma), mastocytoma, and basal cell cancer, and more preferably malignant melanoma (melanoma). In addition, the metastasis inhibition of tumor cells according to the present invention is preferably metastasis inhibition of tumor cells to lung.

Meanwhile, it is known that glycolysis inhibitors such as 2-deoxyglucose (2-DG) compete with glucose and are taken up by cancer cells via GLUT1 and inhibit the glycolytic system of cancer cells, thereby slowing the proliferation rate of cancer cells. Here, according to the study of the present inventors, it has been found that, when the compound represented by the Chemical Formula 1A or Chemical Formula 1B described above (for example, neopetasin) is applied to tumor cells together with glycolysis inhibitors such as 2-deoxy-D-glucose, the compound exhibits an effect of enhancing the action of the glycolysis inhibitors (Test Example 3 to be described later). Therefore, according to one aspect of the present invention, there is also provided an action enhancer of glycolysis inhibitors that contains the compound represented by the Chemical Formula 1A or Chemical Formula 1B described above as an effective component. Similarly, the present invention also provides a method for enhancing the action of glycolysis inhibitors that includes administering the compound according to the present invention described above (preferably, neopetasin) to a patient in need thereof together with a glycolysis inhibitor. Furthermore, according to the present invention, the compound according to the present invention described above (preferably, neopetasin) for use in enhancing the action of glycolysis inhibitors is also provided. Moreover, according to the present invention, application of the compound according to the present invention described above (preferably, neopetasin) to action enhancement of glycolysis inhibitors is also provided. In addition, in the present aspect, the compound according to the present invention (preferably, neopetasin) is used in combination with a glycolysis inhibitor to enhance the action of the glycolysis inhibitor.

Here, the glycolysis inhibitors that can be used together with the effective component according to the present invention is not particularly limited, and a substance having an action of inhibiting a glycolytic pathway (examples include synthetic compounds, peptides, proteins, antibodies, non-peptide compounds, fermentation products, cell extracts, plant extracts, animal tissue extracts, plasma, and the like) itself or an agent containing such substance may be used, and examples thereof include sodium fluoride, lithium iodoacetic acid, and the like, besides the 2-deoxy-D-glucose (2-DG) described above.

According to still another aspect of the present invention, there is provided a pharmaceutical composition for suppression and/or prevention of metastasis of a tumor that contains the compound according to the present invention described above or the metastasis inhibitor of tumor cells according to the present invention described above. Similarly, according to the present invention, there is also provided a pharmaceutical composition for suppression of metastasis of a tumor cell that contains the compound according to the present invention described above (preferably, neopetasin) and pharmaceutically acceptable additives. Furthermore, according to the present invention, there is also provided that contains the compound according to the present invention described above (preferably, neopetasin) for use in suppressing metastasis of tumor cells.

As described above, one aspect of the present invention relates to a pharmaceutical composition for suppression and/or prevention of metastasis of a tumor that contains the compound according to the present invention or the metastasis inhibitor of tumor cells according to the present invention described above. The compound according to the present invention or the pharmaceutical composition for suppression and/or prevention of metastasis of a tumor cell according to the present invention described above can be used for suppressing and/or preventing metastasis of various tumors described above, and is particularly preferably used for suppressing or preventing metastasis of malignant melanoma (melanoma). Moreover, from another viewpoint, the pharmaceutical composition for suppression and/or prevention of metastasis of a tumor cell according to the present invention is preferably used for suppressing and/or preventing metastasis of a tumor cell to lung. Note that "prevention of metastasis of tumor cells" or "prevention of tumor cell metastasis" means preventing or delaying metastasis of cancers or tumors or reducing the risk of cancer or tumor metastasis. Furthermore, as described above, since the effective component according to the present invention has an effect of enhancing the action of glycolysis inhibitors, the pharmaceutical composition according to the present invention is preferably administered in combination with the glycolysis inhibitors.

As a result of further studies conducted by the present inventors for the purpose of clarifying the action mechanisms by which the compound according to the present invention exhibits a proliferation inhibitory activity or a metastasis inhibitory activity on tumor cells, it has been found that the compound according to the present invention has an action of inhibiting aspartic acid synthesis in tumor cells (Test Example 6 and Test Example 7 of the Examples to be described later) . In other words, according to one aspect of the present invention, there is provided an aspartic acid synthesis inhibitor that contains at least one of the compounds according to the present invention described above as an effective component. Similarly, the present invention also provides a method of inhibiting the synthesis of aspartic acid that includes administering a compound according to the present invention as described above (preferably, neopetasin) to a patient in need thereof. Furthermore, according to the present invention, the compound according to the present invention described above (preferably, neopetasin) for use in inhibiting the synthesis of aspartic acid is also provided. Moreover, according to the present invention, an application of the compound according to the present invention described above (preferably, neopetasin) to the inhibition of the aspartic acid synthesis is also provided. From this, it is considered that the compound of the present invention exhibits a proliferation inhibitory activity and a metastasis inhibitory activity on tumor cells by inhibiting the aspartic acid synthesis in tumor cells. In addition, the "inhibition of aspartic acid synthesis" according to the present aspect is preferably for suppressing metastasis of tumor cells (in other words, it is intended to suppress the metastasis of tumor cells).

Furthermore, the present inventors have also found that the compound according to the present invention has an action of inhibiting the spheroid formation in tumor cells (Test Example 4 of the Examples to be described later). In other words, according to one aspect of the present invention, there is provided a spheroid formation inhibitor of tumor cells that contains at least one of the compounds according to the present invention described above as an effective component. Similarly, according to the present invention, there is also provided a method for inhibiting the spheroid formation in tumor cells that includes administering the compound according to the present invention described above (preferably, neopetasin) to a patient in need thereof. In addition, according to the present invention, the compound according to the present invention described above (preferably, neopetasin) for use in inhibiting the spheroid formation in tumor cells is also provided. Furthermore, according to the present invention, an application of the compound according to the present invention described above (preferably, neopetasin) to the inhibition of the spheroid formation in tumor cells is also provided. From this, it is considered that the compound of the present invention exhibits a proliferation inhibitory activity and a metastasis inhibitory activity on tumor cells also by inhibiting the spheroid formation in tumor cells. In addition, the "inhibition of the spheroid formation in tumor cells" according to the present aspect is preferably for suppressing metastasis of tumor cells (in other words, it is intended to suppress metastasis of tumor cells).

Furthermore, for the purpose of clarifying the action mechanisms of the compound according to the present invention for inhibiting the aspartic acid synthesis in tumor cells, the present inventors conducted further studies, and as a result, it was found that the compound of the present invention has an action of inhibiting Complex I constituting the mitochondrial respiratory chain in tumor cells (Test Example 8 and Test Example 9 of the Examples to be described later). Therefore, it is considered that at least one of the action mechanisms by which the compound of the present invention inhibits aspartic acid synthesis in tumor cells is by inhibiting Complex I of the mitochondrial respiratory chain.

Furthermore, according to the study of the present inventors, it was also found that the compound of the present invention has an action of suppressing expression of various cancer-related molecules (proteins) (for example, a phosphorylated (activated) form of Akt, ERK1/2, FAK, or STAT3, or alternatively AMPK, mTOR, ACC, GSK3b, and the like) in tumor cells (Test Example 10 and Test Example 11 of the Examples to be described later) . From this, it is considered that the expression suppression of various cancer-related molecules (proteins) as described above is also involved in the expression of the inhibitory effects on proliferation and metastasis inhibitory function of tumor cells.

As described above, the pharmaceutical composition for suppression and/or prevention of metastasis of a tumor cell according to the present invention contains the metastasis inhibitor of tumor cells according to the present invention. In addition, the metastasis inhibitor of tumor cells according to the present invention includes at least one of the mitochondrial respiratory chain Complex I inhibitor in tumor cells according to the present invention described above, an aspartic acid synthesis inhibitor in tumor cells, an expression inhibitor of phosphorylated (activated) type of Akt, ERK1/2, FAK, or STAT3, or of AMPK, mTOR, ACC or GSK3b in tumor cells, or a spheroid formation inhibitor in tumor cells. Therefore, the pharmaceutical composition for suppression and/or prevention of metastasis of a tumor cell according to the present invention contains the compound represented by the Chemical Formula 1A or Chemical Formula 1B described above as an effective component. The pharmaceutical composition can be used in a dosage form similar to a conventional one. In other words, the pharmaceutical composition according to the present aspect can be used in the form of a pharmaceutical product or the like that is suitable for parenteral administration, oral administration, or external administration by mixing the compound represented by the Chemical Formula 1A or Chemical Formula 1B with pharmaceutically acceptable additives such as an excipient.

Targets to be treated with the pharmaceutical composition is not particularly limited, but is a mammal or a bird, preferably a mammal or a bird suffering from cancer. Here, the mammal includes both primates such as humans, monkeys, gorillas, chimpanzees, and orangutans, and non-human mammals such as mice, rats, hamsters, guinea pigs, rabbits, dogs, cats, pigs, cows, horses, sheep, camels, and goats. Examples of birds include chickens, quails, pigeons, and the like. Among them, humans, hamsters, guinea pigs, rabbits, dogs, cats, and pigs are preferred, and humans as well as pet animals such as dogs, cats, and rabbits are more preferred. In other words, according to a preferred embodiment of the present invention, the pharmaceutical composition for suppression and/or prevention of metastasis of a tumor cell according to the present invention is preferably orally administered to humans or pet animals, and more preferably orally administered to humans or at least one pet animal selected from the group consisting of dogs, cats, and rabbits. According to a more preferred embodiment of the present invention, the pharmaceutical composition for suppression and/or prevention of metastasis of a tumor cell according to the present invention is orally administered to humans, dogs, or cats.

The above pharmaceutical composition can be provided as an oral preparation, an external preparation, an injection, an inhalant, a nasal drop, an eye drop, or the like, and can be formed into desired dosage forms such as a tablet, a liquid preparation, an injection, an ointment, a cream, a lotion, an aerosol, a suppository depending on use methods thereof. In addition, an excipient, a base, an emulsifier, a stabilizer, a dissolution aid, a flavoring agent, a preservative, a fragrance, a coloring agent, a coating agent, and the like can be appropriately blended as necessary.

The dosage of the above pharmaceutical composition is determined in consideration of therapeutically effective amount, but routes of administration vary depending on disease properties of patient, size of patient, weight, surface area, age, as well as sex; other agents administered; judgment of attending physician, or the like. Suitable dosages are, for example, 1 to 500 mg/kg body weight per day as an effective component. In consideration of efficacy variation due to various routes of administration of the above various pharmaceutical compositions available, it is expected that the dosage required may vary widely. Variations in these dosage levels can be adjusted using standard empirical procedures with known optimality in the art. Particularly for oral delivery, delivery efficiency can be increased by encapsulating the above pharmaceutical composition in a suitable delivery vehicle (for example, polymeric microparticles or implantable devices). Moreover, the above dosage may be divided once or a plurality of times a day. Furthermore, in some cases, it may be administered at a lower frequency (for example, on a weekly or monthly basis). In addition, even for the same patient, the dosage may vary depending on the patient's symptoms and severity.

In the present description, a "therapeutically effective amount" means, when used in the present description, an amount of the effective component or pharmaceutical composition that is effective to produce any desired inhibitory effect at a reasonable benefit/risk ratio applicable to any medical treatment. For example, the dosage of the above pharmaceutical composition according to the present invention varies depending on target diseases, the targets to be administered, the routes of administration, and the like, but when the above pharmaceutical composition is orally administered, since the dosage can be easily changed depending on conditions such as body weight of the subject, the dosage can be appropriately selected by those skilled in the art. Furthermore, finally, the attending physician appropriately selects the dosage in consideration of the patient's symptoms, severity, and the like.

In the present description, "pharmaceutically acceptable" is used to refer to compounds, materials, compositions, and/or dosage forms that are, commensurate with a reasonable benefit/risk ratio within the scope of sound medical judgment, suitable for use in contact with tissues of targets to be treated (humans, mammals, or the like) without problems or complications such as excessive toxicity, irritation, and allergic response.

Pharmaceutically acceptable carriers means a pharmaceutically acceptable material, composition or excipient, such as a liquid or solid filler, diluent, excipient, solvent, or encapsulating material that is involved in carrying or transporting an agent for suppressing/preventing cancer metastasis of the reproductive system of the present invention from one organ or part of the body to another organ or part of the body. Each carrier should be "acceptable" in the sense of being compatible with the other components of the dosage form and not harmful to the patient. Pharmaceutically acceptable carriers include, but are not limited to, sugars such as lactose, glucose and sucrose; starches such as corn starch and potato starch; celluloses and derivatives thereof such as sodium carboxymethylcellulose, ethylcellulose, and cellulose acetate; tragacanth powder; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline solution; ringer's solution; ethyl alcohol; phosphate buffer solution; and other non-toxic compatible substances used in drug formulations. In some embodiments, the drug formulation is non-pyrogenic. In other words, it is desirable not to raise body temperature of the patient. In addition, wetting agents such as sodium lauryl sulfate and magnesium stearate, emulsifiers and lubricants, and coloring agents, releasing agents, covering agents, sweeteners, flavoring agents and aromatics, preservatives and antioxidants may be included in the agents for suppressing/preventing cancer metastasis of the reproductive system of the present invention.

Pharmaceutically acceptable antioxidants include, but are not limited to, water-soluble antioxidants such as ascorbic acid, cysteine hydrochloride, sodium hydrogen sulfate, sodium disulfite, and sodium sulfite; oil-soluble antioxidants such as ascorbyl palmitate, butylated hydroxyanisole (BHA), butylated hydroxytoluene (BHT), lecithin, propyl gallate, α-tocopherol; and metal chelating agents such as citric acid, ethylenediaminetetraacetic acid (EDTA), sorbitol, tartaric acid, and phosphoric acid.

The pharmaceutical composition according to the present invention can be used in various dosage forms such as oral, nasal, topical (including buccal and sublingual), rectal, vaginal and/or parenteral administration, but is preferably oral administration. Dosage forms may conveniently be presented in unit dosage form and may be prepared by any method well known in the pharmaceutical art. The amount of active component that can be combined with carrier material to make a single dosage form would vary depending on host to be treated and particular mode of administration. The amount of active component that can be combined with the carrier material to make a single dosage form is generally the amount of the compound that produces a therapeutic effect, but generally, the amount of effective component (the compound represented by Chemical Formula 1A or Chemical Formula 1B) is from about 0.1 to about 99 wt%, preferably from about 1 to about 70 wt%, and more preferably from about 5 to about 50 wt%, based on total amount of the pharmaceutical composition.

The process for preparing these dosage forms or compositions includes a step of linking the effective component according to the present invention with a carrier, optionally with one or more accessory components. In general, the dosage forms are prepared by uniformly and intimately linking one or more effective components according to the present invention to a liquid carrier, or a finely divided solid carrier, or both, and shaping the product if necessary.

For example, the dosage forms suitable for oral administration may be in the form of capsules, sachets, pills, tablets, lozenges (using a flavored principal agent, usually sucrose and gum arabic or tragacanth), powders, granules, or as solutions or suspensions in aqueous or non-aqueous liquids, or as oil-in-water or water-in-oil liquid emulsions, or as elixirs or syrups, or as pastilles (using gelatin and glycerin, or inert bases such as sucrose and gum arabic) and/or gargling agents and the like, each containing a predetermined amount of compound of the effective component according to the present invention as an active component. The pharmaceutical composition according to the present invention may be administered as a large pill, electuary, or paste.

In solid dosage forms (capsules, tablets, pills, dragees, powdered medicines, granules, and the like) for oral administration, the effective component is mixed with one or more pharmaceutically acceptable carriers such as sodium citrate or dicalcium phosphate, and/or any of the following: fillers or bulking agents such as starch, lactose, sucrose, glucose, mannitol, and/or silicic acid; for example, binders such as carboxymethylcellulose, alginate, gelatin, polyvinylpyrrolidone, sucrose and/or gum arabic; humectants such as glycerol; disintegrants such as agar, calcium carbonate, potato or tapioca starch, alginic acid, certain silicates, and sodium carbonate; lysis retarder such as paraffin; absorption enhancers such as quaternary ammonium compounds; wetting agents such as cetyl alcohol and glycerol monostearate; absorbents such as kaolin and bentonite clay; lubricants such as talc, calcium stearate, magnesium stearate, solid polyethylene glycol, sodium lauryl sulfate, and mixtures thereof; and coloring agents. In the case of capsules, tablets and pills, these dosage forms may contain buffering agents. Solid compositions of the same kind can also be used as fillers in soft and hardfilled gelatin capsules with excipients such as lactose or milk sugar, high molecular weight polyethylene glycols, and the like.

Furthermore, tablets may be made by compression or molding, optionally with one or more accessory components. Compressed tablets may be prepared with binders (for example, gelatin or hydroxypropyl methylcellulose), lubricants, inert diluents, preservatives, disintegrants (for example, sodium starch glycolate or cross-linked sodium carboxymethylcellulose), surfactants, or dispersants. Molded tablets may be made by molding a mixture of powder compounds wetted with an inert liquid diluent in a suitable machine.

Solid dosage forms such as tablets of the pharmaceutical composition according to the present invention like dragees, capsules, pills, and granules, may optionally be scored or prepared with coatings and shells such as enteric coatings well known in the drug formulating art. They may be formulated to provide a sustained or controlled release of the effective component therein using, for example, hydroxypropyl methylcellulose, other polymeric matrices, liposomes and/or microspheres in various ratios in order to provide a desired release profile. They may be sterilized, for example, by filtration through a bacteria-retaining filter or by incorporating sterilizing agents in the form of a sterile solid composition that can be dissolved in a sterile injectable medium such as sterile water immediately prior to use. These compositions may optionally contain opacifiers and may be compositions that release one or more effective components only in certain portions of the gastrointestinal tract, or preferentially, optionally, in a delayed manner. Examples of embedding compositions that may be used are polymeric substances and waxes. The effective component may be in microencapsulated form, if appropriate, with one or more excipients as described above.

Liquid dosage forms for oral administration of the pharmaceutical composition according to the present invention include pharmaceutically acceptable emulsions, microemulsions, solutions, suspensions, syrups and elixirs. Liquid dosage forms may include, in addition to the active component, inert diluents commonly used in the art such as, for example, water and other solvents, solubilizers and emulsifiers such as ethyl alcohol, isopropyl alcohol, ethyl carbonate, ethyl acetate, benzyl alcohol, benzyl benzoate, propylene glycol, 1,3-butadiene glycol, oils (particularly cottonseed oil, peanut oil, corn oil, embryo oil, olive oil, castor oil and sesame oil), glycerol, tetrahydrofuryl alcohol, polyethylene glycols, and fatty acid esters of sorbitan, and mixtures thereof. Furthermore, in addition to the inert diluents, oral compositions may also contain adjuvants such as the wetting agents, emulsifiers and suspending agents, sweeteners, flavoring agents, coloring agents, aromatics and preservatives. Suspensions may contain, in addition to active compounds, suspending agents such as, for example, ethoxylated isostearyl alcohols, polyoxyethylene sorbitol and sorbitan esters, microcrystalline cellulose, aluminum metahydroxide, bentonite, agar and tragacanth, and mixtures thereof.

Dosage form of the pharmaceutical composition according to the present invention for rectal or vaginal administration may be presented as a suppository. This suppository can be prepared by mixing one or more suitable non-irritating excipients or carriers, including, for example, cocoa butter, polyethylene glycol, suppository wax or salicylate, with one or more effective components of the present invention, which are solid at room temperature but liquid at body temperature and melts in rectum or vaginal cavity to release the active compounds. Suitable dosage forms for vaginal administration also include pessaries, tampons, creams, gels, pastes, foams or spray dosage forms that contain carriers known to be suitable in the art.

Dosage forms for topical or transdermal administration of the pharmaceutical composition according to the present invention include powders, sprays, ointments, pastes, creams, lotions, gels, solutions, patches, and inhalants. The effective component may be mixed under sterile conditions with a pharmaceutically acceptable substrate and, if necessary, preservatives, buffering solutions, or propellants. Ointments, pastes, creams and gels may contain, in addition to the effective component, excipients such as animal fat or vegetable fat, oils, waxes, paraffins, starches, tragacanths, cellulose derivatives, polyethylene glycols, silicones, bentonites, silicic acids, talcs, and zinc oxides, or mixtures thereof.

The powders and sprays may contain, in addition to the effective component, excipients such as lactose, talc, silicic acid, aluminum hydroxide, calcium silicate, and polyamide powder, or mixtures of these substances. The sprays may further contain customary high pressure gases such as fluorinated chlorinated hydrocarbons and volatile unsubstituted hydrocarbons such as butane and propane.

Transdermal patches have an additional advantage of controlled delivery of the pharmaceutical composition according to the present invention to the body. Such dosage form can be made by dissolving or dispersing the effective component according to the present invention in a suitable medium. It is also possible to increase a flux of a substance containing the effective component according to the present invention across the skin using an absorption enhancer. The speed of such fluxes can be controlled either by providing a speed-control membrane or by dispersing the compound in a polymer matrix or gel.

The pharmaceutical composition according to the present invention suitable for parenteral administration contains, together with the composition, one or more pharmaceutically acceptable sterile isotonic aqueous or non-aqueous solutions, dispersants, suspensions or emulsions, or sterile powders that can be returned in sterile injectable solutions or dispersions shortly before use, and such sterile powders may contain antioxidants, buffering agents, bacteriostats, solutes which render the preparation isotonic with blood of intended recipient, or suspending agents or concentrates.

Examples of suitable aqueous and non-aqueous carriers that can be used in the pharmaceutical composition according to the present invention include water, ethanol, polyols (for example, glycerol, propylene glycol, polyethylene glycol, and the like), and suitable mixtures thereof, vegetable oils such as olive oil, and injectable organic esters such as ethyl oleate. The inherent fluidity can be maintained, for example, by use of covering materials such as lecithin, by maintenance of the required particle size in the case of dispersants, and by use of surfactants.

The pharmaceutical composition according to the present invention may contain adjuvants such as preservatives, wetting agents, emulsifiers and dispersants. Prevention of microbial activity may be ensured by containing various antibacterial and antifungal agents, such as, for example, parabens, chlorobutanol, phenol sorbate. It may be desirable to contain isotonic agents such as sugars, sodium chloride in the composition. Furthermore, persistent absorption of injectable drug form may be caused by containing agents that delay absorption, such as aluminum monostearate and gelatin.

Still another embodiment of the present invention relates to a method for suppressing and/or preventing metastasis of tumor cells, including administering the pharmaceutical composition according to the present invention to a patient in need of suppressing the metastasis of tumor cells. Furthermore, one preferred embodiment relates to a method for suppressing and/or preventing the metastasis of tumor cells after cancer resection, including administering the pharmaceutical composition according to the present invention to a patient in need of suppressing the metastasis of tumor cells after cancer resection.

According to still another embodiment of the present invention, there is provided a food and drink composition for suppressing the metastasis of tumor cells, containing the compound represented by Chemical Formula 1A or Chemical Formula 1B described above as an effective component.

The form of the food and drink composition for suppressing the metastasis of tumor cells is not particularly limited, and the food and drink composition may be added to a liquid, gel or solid food (for example, juice, soft drink, tea, soup, soy milk, salad oil, dressing, yogurt, jelly, pudding, Furikake, powdered milk for child care, cake mix, powdered or liquid dairy product, bread, cookies, and the like), or may be processed into pellets, tablets, granules, or the like together with excipients such as dextrin, lactose, or starch, a fragrance, a pigment, or the like as necessary. In addition, at least one of the compounds represented by Chemical Formula 1A or Chemical Formula 1B described above can be coated with gelatin or the like and molded into a capsule to be used as a health food, a nutritional supplement, or the like. It is difficult to uniformly define the blending amount of the above compound (the effective component) contained in the food and drink composition for suppressing the metastasis of tumor cells depending on types, states, and the like of the food and composition, but it is 0.01 to 90 wt%, more preferably 0.1 to 80 wt% with respect to total weight of the food. With such a blending amount, the efficacy of the above compound (the effective component) can be sufficiently exhibited without impairing the flavor. In addition, food can be easily prepared.

Here, the "food and drink composition" means a composition intended for ingestion by mammals (including pets) such as a human. For example, pet food composition is a food and drink composition intended for ingestion by pets. The food and drink compositions are widely known in the art. The pet food composition may or may not be nutritionally balanced and may be a nutritionally balanced composition suitable for everyday meals besides supplements (for example, feed). Here, "nutritionally balanced" means that the composition of the present invention has the known nutrients necessary for sustaining life, in appropriate amounts and proportions in the view of pet nutrition profession.

In one preferred embodiment of the present invention, the food and drink composition for suppressing metastasis of tumor cells is a human health food or a pet food composition.

Here, when the compound (the effective component) represented by Chemical Formula 1A or Chemical Formula 1B according to the present invention is added to and used in a health food for humans (a human health food) or a pet food composition, the addition amount (the content) of the compound (the effective component) is not particularly limited, but is preferably about 0.01 to 30 wt%, more preferably about 0.05 to 20 wt% with respect to the health food or the pet food composition (in terms of solid content). Alternatively, the amount of the compound is such that the above compound is administered in an amount of preferably about 0.1 to 1000 mg/kg body weight, more preferably about 1 to 500 mg/kg body weight as a total weight per day. With such an amount, a sufficient metastasis inhibitory effect on tumor cells can be achieved. In addition, when the amount is of as described above, the preference of a pet is not inhibited, and the pet ingests the total amount of the given pet food.

The pet food composition contains the same components as a conventional one except that it contains the effective component according to the present invention. For example, the pet food composition may contain, in addition to the effective component according to the present invention, carbohydrate sources such as monosaccharide, oligosaccharide, polysaccharide, dietary fiber, starches (for example, waxy corn starch, corn starch, wheat starch, rice starch, glutinous rice starch, potato starch, sweetened starch, tapioca starch, sago starch, starches obtained by subjecting these substances to chemical treatment, or chemically modified processed starches), or grains (for example, corn, barley, wheat, rye, sorghum, rice, barnyard millet, millet, Amarasanthus, and quinoa); animal protein sources such as animal meat and butcher meat including cattle, pigs, sheep, rabbits, and kangaroos, by-products and processed products thereof, bird meat including chicken, turkey, and quail, by-products and house products thereof, fish meat including fish and white-meat fish, by-products and processed products thereof, and lettering of the above raw materials including meat meal, meat bone, chicken meal, portable meal, and fish meal; plant-derived protein sources such as soybean protein, wheat protein, wheat gluten, and corn gluten; plant sterols such as free forms including α-sitosterol, β-sitosterol, stigmasterol, campesterol, α-sitostanol, β-sitostanol, stigmastanol, campestanol, and cycloartenol, and ester forms including fatty acid esters, ferulic acid esters, and cinnamic acid esters thereof; brans including rice brans and heat brans, cakes including soybean cake, vegetables including vegetable extract, and vitamins including vitamins A, B1, B2, D, and E, niacin, pantothenic acid, and carotene, or the like. In addition to the above, other additives such as gelling agents, mold retention agents, pH adjusting agents, seasonings, preservatives, and nutrient reinforcing agents that are generally used in the pet food composition may be contained. In addition to or in place of the above other additives, antioxidizing agents such as butylhydroxyanisole (BHA), dibutylhydroxytoluene (BHT), ethoxyquine, tertbutylhydroquinone (TBHQ), and propyl gallate can be used in combination. The addition amount (content) of each component described above is not particularly limited, and the same amount as in the conventional art can be applied.

The pet food composition is produced through a conventionally known method. For example, it can be produced by mixing the effective component according to the present invention and the necessary components mentioned above to make a desired form. As an example, citric acid or citrate is mixed, if necessary, together with the effective components described above, grains, meat meal, and mineral components such as iron and copper, and after sufficient mixing, extrusion molding is performed by an extruder while water or water vapor is added thereto. Thereafter, hot air drying is performed until the moisture content is preferably 10% or less, thereby producing the pet food composition. In addition, when the pet food composition contains fat and oil with fatty acid having two or more double bonds, it is desirable to perform coating after hot-air drying.

The pet food may be in any form such as a dry type, a wet type, a semi-moist type, a jerky type, a biscuit type, a gum type, a granular type, a powdery type, or a soup type, but the pet food is preferably a dry type from the viewpoint of convenience of storage. Examples of dry-type pet hood include a kibble shape, a flat plate shape, a bone shape, and the like. From the viewpoint of whether an ease of chewing and a shape easy to handle for a pet can be obtained, it is preferable that bulk density is 100 kg/m³ or more, preferably 300 kg/m³ or more, and 900 kg/m³ or less, preferably 700 kg/m³ or less, or 100 to 900 kg/m³, particularly 300 to 700 kg/m³. Also, the pet food may be provided in bagged, boxed, packed, canned, retortpouched form.

### Examples

Hereinafter, examples of the present invention will be described, but the present invention is not limited to these examples at all.

### (Test Example 1)

Four compounds of petasin, S-petasin, neopetasin and neo-S-petasin were evaluated for the inhibitory effect on proliferation of mouse or canine malignant melanoma cells by the following method.

B16F10 cells (mouse malignant melanoma cells) were obtained from JCRB Cell Bank (National Institutes of Biomedical Innovation, Health and Nutrition). In addition, CMeC1 cells (canine malignant melanoma cells) were obtained from Veterinary Surgery Laboratory, Graduate School of Agricultural and Life Sciences, the University of Tokyo. Furthermore, A2058 cells (human malignant melanoma cells) were obtained from JCRB Cell Bank (National Institutes of Biomedical Innovation, Health and Nutrition). B16F10 cells were cultured in a 37°C incubator (air/CO₂ = 95/5 (v/v)) using Dulbecco's modified Eagle's medium (DMEM), CMeC1 cells in RPMI (Roswell Park Memorial Institute) medium (RPMI1640), and A2058 cells in E-MEM medium.

First, CMeC1 cells (canine malignant melanoma cells) were seeded in a six-well plate so as to be 0.5×10⁵ cells/mL, and after 24 hours therefrom, each of the above four kinds of compounds (final concentration: 0.01 to 100 µg/mL) was suspended in the medium at a ratio of 0.1% and incubated for 72 hours. 72 hours after the start of culture, the number of viable cells was measured by trypan blue staining. At this time, viable cells (cell viability%) were calculated as the relative number of cells when the number of viable cells in a negative control section (DMSO treatment) was taken as 100%. Then, IC₅₀ was calculated using a GraphPad Prism software system (GraphPad Software Inc.).

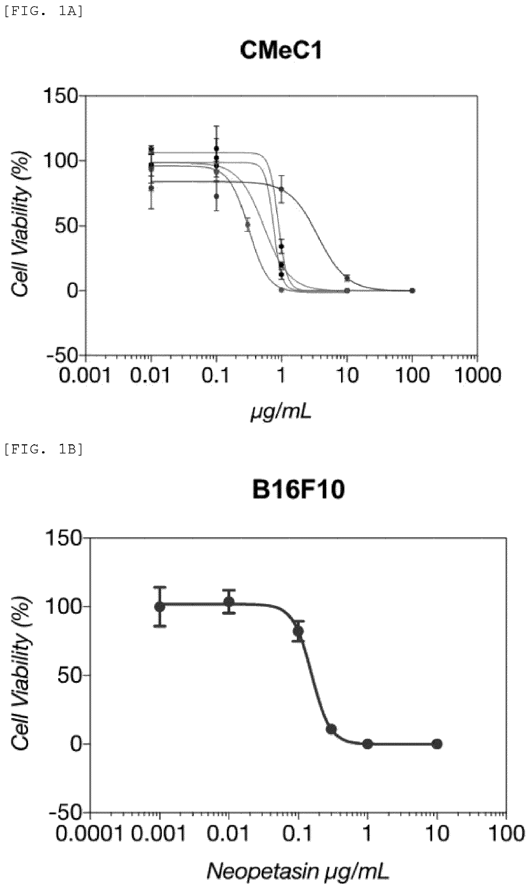

The results are shown in Fig. 1A and Table 1 below. As illustrated in Fig. 1A and Table 1, all of the above four kinds of compounds exhibited the inhibitory effect on proliferation of CMeC1 cells, and among them, neopetasin showed the strongest activity (IC₅₀ = 0.99 µM (0.314 µg/mL)) .

**[Table1]**

| Cell line: CMeC1 | | | |
|---|---|---|---|
| **Compound** | **IC₅₀ (µg/mL)** | **IC₅₀ (µM)** | **g/mol** |
| Fukinoto | 3.624 | - | |
| Petasin | 0.890 | 2.814 | 316.435 |
| Neopetasin | 0.314 | 0.991 | 316.441 |
| S-petasin | 0.755 | 2.257 | 334.474 |
| Neo-S-petasin | 0.537 | 1.605 | 334.474 |

Furthermore, Fig. 1B shows the results of a similar experiment on neopetasin using B16F10 cells (mouse malignant melanoma cells). As illustrated in Fig. 1B, neopetasin showed strong proliferation inhibitory activity also on B16F10 cells (IC₅₀ = 0.493 µM (0.156 µg/mL)). It is found that the proliferation inhibitory activity of such neopetasin is extremely low, and the proliferation inhibitory activity is extremely high as compared with the activity of a classical anticancer agent (the activity of cisplatin (IC₅₀ for B16F10 cells is 5 µg/mL).

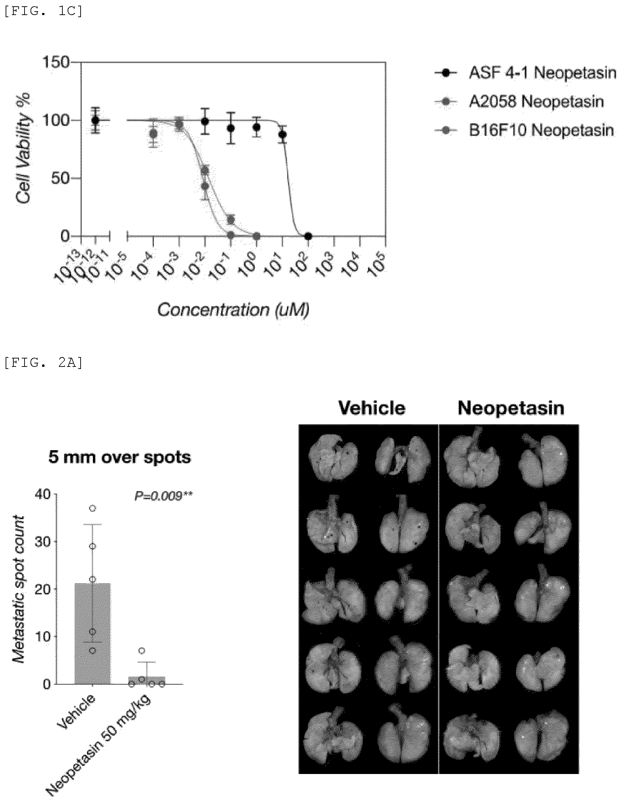

Furthermore, the results of the same test as described above using ASF4-1 cells, which are normal human skin-derived fibroblasts, and A2058 cells, which are human malignant melanoma cells, are shown in Fig. 1C in comparison with the case of using B16F10 cells. As illustrated in Fig. 1C, the IC₅₀ value of neopetasin for ASF4-1 cells (normal cells) was 1000 fold or more larger than the IC₅₀ values for tumor cells, A2058 cells and B16F10 cells. From this, it can be seen that neopetasin hardly exhibits the inhibitory effect on proliferation of normal cells and selectively exhibits the inhibitory effect on proliferation of tumor cells.

### (Test Example 2)

Using the mouse malignant melanoma lung metastasis model, the metastasis inhibitory effect of neopetasin on tumor cells was examined. Specifically, C57BL/6 mice (6 weeks old, female) were purchased from Japan SLC (Hamamatsu). Then, the B16F10 cells were intravenously administered from tail vein in an amount of 0.25×10⁶ cells/100 µL PBS. Then, from the day when the cells were seeded, neopetasin (50 mg/kg) was intraperitoneally administered once every two days (5 times in total). After 10 days from the start of administration, the lungs were dissected and extracted, and the number of metastasis lesions observed on the lung surface was counted. In addition, the P value was calculated using Student's T-test using the GraphPad Prism software system (GraphPad software Inc) .

The results are shown in Fig. 2A. In Fig. 2A, "Neopetasin" means a neopetasin administration group (neopetasin suspensed in corn oil), and "Vehicle" indicates a negative control section (corn oil administration group). The results shown in Fig. 2A indicate that the neopetasin treatment extremely potently suppressed lung metastasis in the mouse malignant melanoma lung metastasis model.

Furthermore, when the model mouse to which neopetasin or corn oil was administered was dissected in the above, sections were prepared from the tissues of each organ (heart, normal site of lung, cerebrum, kidney, liver, spleen) and were observed using a microscope. The results are shown in Fig. 2B. As illustrated in Fig. 2B, no histological and morphological changes after neopetasin administration was observed. The results are consistent with specificity of the action of neopetasin on tumor cells in vitro as described above in Test

### Example 1.

### (Test Example 3)

2-deoxyglucose (2-DG), which is a glycolysis inhibitor, competes with glucose and is taken up by cancer cells via GLUT1, and is known to reduce the proliferation rate of cancer cells by inhibiting the glycolytic pathway of cancer cells. In this test example, the influence of neopetasin on the anticancer action of such 2-DG on tumor cells was examined.

Specifically, to start with, B16F10 cells were seeded in a six-well plate in an amount of 0.5×10⁵ cells/mL. Next, after 24 hours starting from seeding, 2-DG (concentration changed from 3 mM to 3 nM) was added and simultaneously treated with neopetasin at a concentration of 0.3 µM or 3 µM. Then, after 36 hours starting from this treatment, the cell viability was measured through the same method as in Test Example 1. Moreover, as a negative control section, DMSO was administered to perform the same experiment.

The results are shown in Fig. 3. As illustrated in Fig. 3, in the negative control section, it was confirmed that the cell viability was reduced in a concentration-dependent manner due to the action of 2-DG. Furthermore, it is found that the anticancer action of such 2-DG was also enhanced in a concentration-dependent manner due to the addition of neopetasin. Since it exhibits a synergistic effect with 2-DG that is a glycolysis inhibitor, it is considered that neopetasin exhibits an anticancer activity by inhibiting the mitochondrial respiratory chain Complex I. Furthermore, it was shown that neopetasin also has an effect of enhancing the effect of an existing cancer metabolism inhibitor such as 2-DG, of which the activity is conventionally low and the anticancer activity could not be exhibited. As described above, by actually conducting experiments, the inventors of the present application have found for the first time that the predetermined compound of the present application such as neopetasin enhances the action of glycolysis inhibitors such as 2-DG. Furthermore, such an effect is remarkable that a person skilled in the art could not predict, and indicates that the present invention could not be obviously derived from the prior art. That is, according to the study of the present inventors, it has been found that neopetasin suppresses the mitochondrial Complex I (Test Example 8 and Test Example 9), and induces inhibition of oxidative phosphorylation (Test Example 10) and further inhibition of aspartic acid synthesis (Test Example 6 and Test Example 7). Moreover, as a result, since production of ATP in the cells is extremely reduced, the cells attempt to maintain the production of ATP by dominantly operating the glycolytic pathway. In other words, it is considered that, when neopetasin acts on cells, the cells enter into a glycolytic pathway dependent state. For the purpose of proving this fact, the present inventors conducted an experiment using 2-DG, which is a glycolysis inhibitor, and demonstrated that sensitivity of cells to 2-DG is actually increased, thereby proving the above fact (that cells are changed into the glycolytic pathway dependent state by the action of neopetasin). From this, it can be said that the present inventors have found, as a result of a series of experiments described above, the fact that "neopetasin enhances action of glycolysis inhibitors such as 2-DG (in other words, the action of neopetasin increases the sensitivity of cells to glycolysis inhibitor)" secondarily. Naturally, it is obvious that the sensitivity of cells to glycolysis inhibitor is improved by the action of neopetasin although the dependence on glycolytic pathway varies depending on cells. Furthermore, due to discovery of this fact, the present invention can provide a new technique of enhancing the action of glycolysis inhibitors by using neopetasin in combination with glycolysis inhibitors.

### (Test Example 4)

The spheroid formation assay was performed on B16F10 cells in the presence of neopetasin to examine an influence of neopetasin on the spheroid formation ability of the cells.

Specifically, B16F10 cells were seeded in an amount of 0.3×10⁵ cells/mL in a 96-well plate for spheroid formation in an amount of 100 µL each. Thereupon, neopetasin at a concentration of 0.3 µM or 3 µM was added to the medium. Cells were photographed using a phase-contrast microscope after 24 hours, 48 hours and 72 hours of adding neopetasin. Moreover, as a negative control section, DMSO was administered to perform the same experiment.

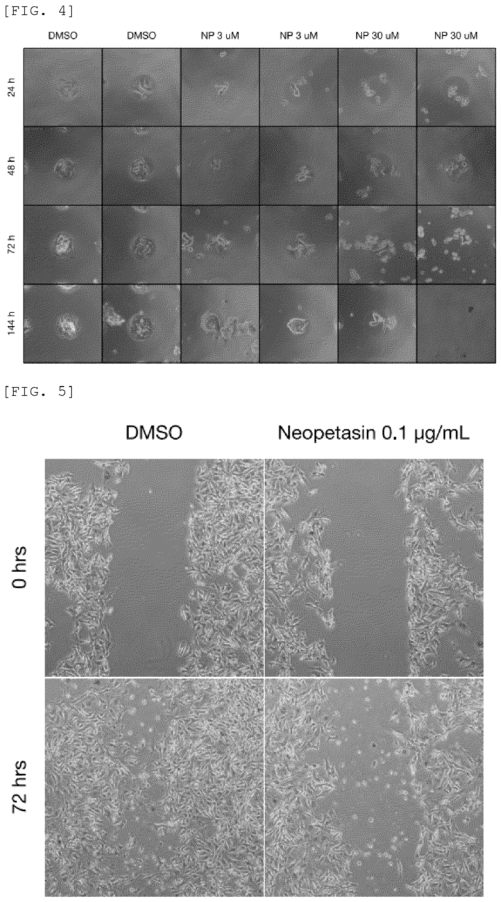

The results are shown in Fig. 4. As illustrated in Fig. 4, the spheroid formation was observed in the negative control section, but it was found that the cells treated with neopetasin could not form spheroids.

### (Test Example 5)

In order to examine the influence of neopetasin on the migration action of tumor cells, the scratch wound healing assay was performed.

Specifically, to start with, B16F10 cells were seeded in a six-well plate in an amount of 1.0×10⁵ cells/mL. After 24 hours starting from seeding, scratches were then made using 200 µL chips. Thereafter, the medium was changed to a neopetasincontaining one (concentration: 0.3 µM), the cells were further cultured for 72 hours, and the cells were photographed. In addition, as a negative control section, the same experiment was performed by replacing the medium with the same medium not containing neopetasin.

The results are shown in Fig. 5. As illustrated in Fig. 5, in the negative control section, it was observed that the tumor cells migrated to a scratch portion after 72 hours of culture. On the other hand, it is found that the migration of tumor cells to the scratch portion is suppressed by neopetasin treatment.

Note that Non-Patent Literature 2 described above discloses that, in addition to inhibiting the proliferation of colon cancer cell lines, the petasin suppressed the migration and invasion of colon cancer cell lines. However, even when it is found that a certain compound suppresses the migration and invasion of tumor cells, whether or not the compound can actually suppress metastasis of tumor cells can only be demonstrated by actually conducting experiments.

### (Test Example 6)

For the purpose of clarifying the action mechanism by which neopetasin exhibits proliferation inhibitory activity and metastasis inhibitory activity on tumor cells, the influence of neopetasin on mitochondria of B16F10 cells was evaluated. The signaling pathways affected by neopetasin treatment were examined by KEGG Pathway analysis using the microarray. Specifically, A2058 cells were seeded at 0.5×10⁵ cells/mL in a six-well plate, and after 24 hours of seeding, neopetasin (final concentration: 3 µM) was suspended in a medium and incubated for 8 hours. A2058 cells thus treated with neopetasin (3 µM) for 8 hours were recovered with a cell scraper. Furthermore, DMSO was administered to the negative control section, and the same experiment was performed. Next, data was collected using SurePrint G3 Human CGH microarray 8x60K (Agilent, CA, USA) and the raw data obtained for each sample was normalized with 75 percentile values (global normalization). Then, using Gene Set Enrichment Analysis (GSEA, Broad Laboratory, Massachusetts Institute of Technology, MA, USA), pathways registered in KEGG, Reactome, and GO were searched.

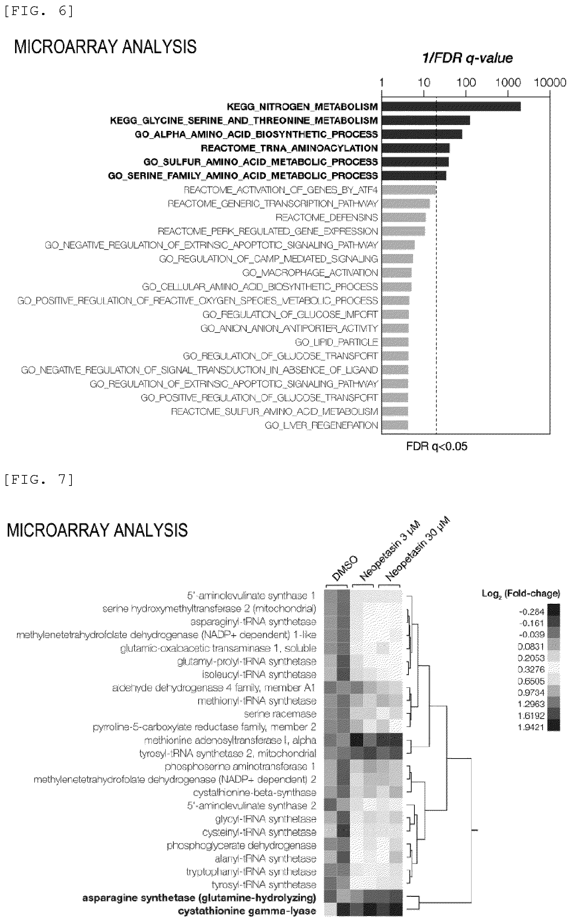

The results are shown in Fig. 6. Fig. 6 illustrates pathways whose q-value of false discovery rate (FDR) are q < 0.25 among the pathways hit in the above search. Furthermore, q-value was q < 0.05 in the six pathays shown from the top in Fig. 6, which showed particularly significant variation. From the results shown in Fig. 6, it is found that the expression of molecules involved in amino acid metabolism greatly varies due to the neopetasin treatment.

Furthermore, Fig. 7 illustrates a heat map that shows a gene having a particularly large variation among the variable pathways shown in Fig. 6. In Fig. 7, the genes with increased expression are shown in red, the gene groups with decreased expression are shown in blue, and the gene groups with changes therebetween are shown in white. As illustrated in Fig. 7, among the molecules contained in the variable pathways, particularly significant changes in asparagine synthetase (ASNS) and cystathionine gamma-lyase (CTH) were observed. In addition, when the heat map was drawn, gene groups included in the variable pathways were extracted, and the log-transformed signal data was subjected to cluster analysis by Ward's method using JMP version 12.2.0 (SAS Institute Inc.).

### (Test Example 7)

The metabolomic analysis (amino acids) of neopetasin-treated B16F10 cells was performed. Specifically, for B16F10 cells treated with neopetasin (3 µM) through the same method as in Test Example 1, the cells were recovered at each time point of 9 hours and 48 hours after the treatment, and amino acid concentration was measured using a capillary electrophoresistime of flight mass spectrometer (CE-TOFMS). Furthermore, the amino acid concentration in the sample was measured according to an internal standard calibration method, and the internal standard concentration in the sample was calculated as 25 µM. Moreover, for the analysis, Master Hands ver. 2.17.3.18 (Institute for Advanced Biosciences, Keio University) was used.

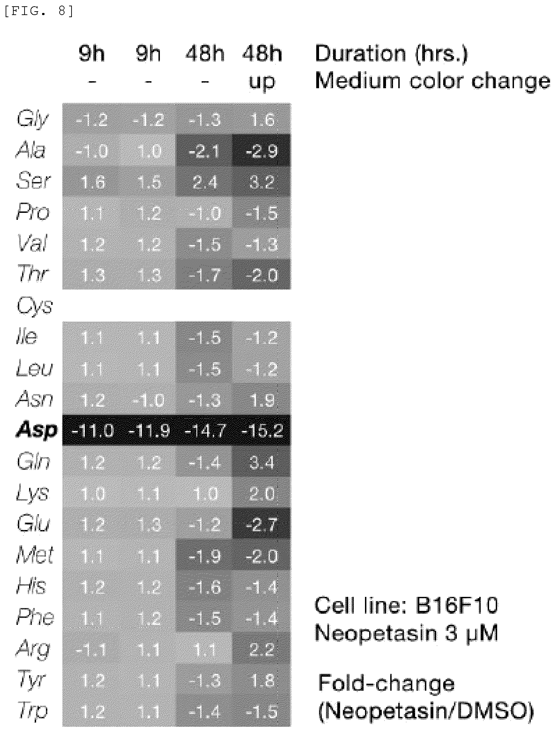

The results are shown in Fig. 8. In Fig. 8, changes in the amino acid concentration contained in the sample is calculated as fold-change and shown as a heat map. Then, the higher the concentration in the sample is, the deeper the red color is, the lower the concentration is, the deeper the blue color is, and the sample below the detection limit is indicated as a blank. From the results shown in Fig. 8, it is found that the neopetasin treatment already strongly reduced the concentration of aspartic acid (Asp) in the sample after 9 hours of neopetasin treatment.

### (Test Example 8)

In order to determine the action mechanism of neopetasin on inhibiting the aspartic acid synthesis in tumor cells, the influence of neopetasin on mitochondria in B16F10 cells was evaluated. Specifically, B16F10 cells treated with neopetasin (3 µM) for 48 hours through the same method as in Test Example 1 were stained using MitoTracker Orange (Thermo Fisher Scientific, MA, USA), which labels mitochondria depending on membrane potential. In addition, as a positive control section, in place of neopetasin, metformin known as a mitochondrial respiratory chain Complex I inhibitor (mitochondrial toxin) was used at a concentration of 5000 µM to perform the same experiment.

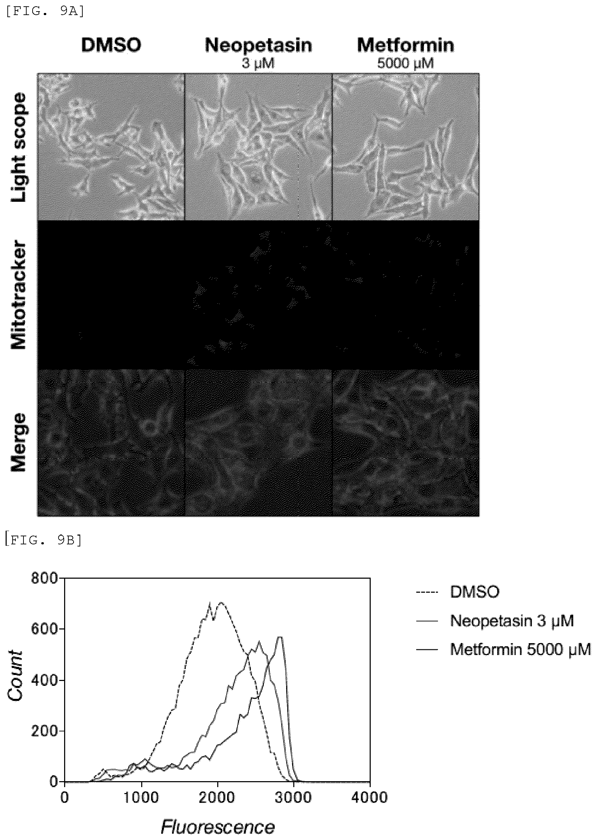

Subsequently, the cells stained as described above were photographed with a fluorescence microscope (Fig. 9A). In addition, the relationship between fluorescence intensity of the cells and the distribution of the number of cells was measured using an image cytometer (Tali, Thermo Fisher Scientific, MA, USA) (Fig. 9B).

As illustrated in Figs. 9A and 9B, enhanced fluorescence intensity (transition to stronger fluorescence peak) was observed in both of the cells treated with the compounds of neopetasin (3 µM) and metformin (5000 µM). From this, it was suggested that these compounds affect the number of mitochondria or the membrane potential. In addition, the activity of neopetasin is 1000 times or more higher than that of metformin.

Following the above results, the cells respectively treated with neopetasin (3 µM) and metformin (5000 µM) in the above were observed using an electron microscope. Furthermore, DMSO was administered to the negative control section, and the same experiment was performed.

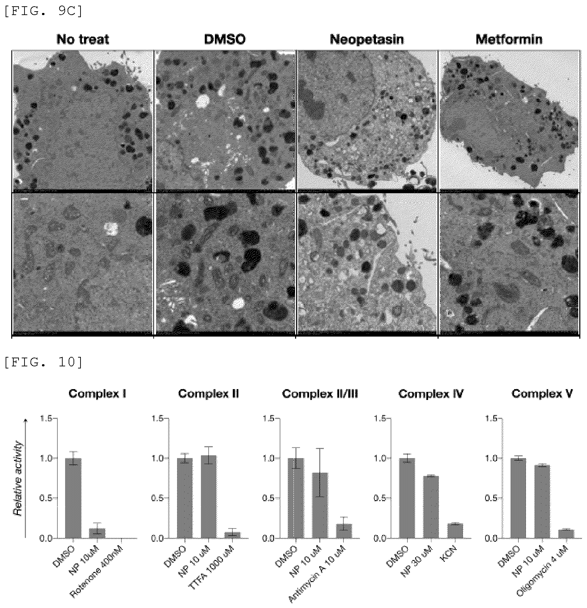

The results are shown in Fig. 9C. As illustrated in Fig. 9C, most mitochondria were vacuolated in the cells treated with neopetasin (3 µM), and it was demonstrated that mitochondria were greatly damaged. On the other hand, in metformin-treated cells, the influence on mitochondria was mild although the cells were treated at an extremely high concentration of 5000 µM. These results suggest that the inhibitory action of neopetasin on the aspartic acid synthesis in tumor cells is due to the action of neopetasin as an extremely potent mitochondrial toxin.

### (Test Example 9)

In order to determine the action points of neopetasin as a mitochondrial toxin, the influence of neopetasin on the activity of each of the complexes (Complexes I to V) constituting the mitochondrial respiratory chain was examined.

Specifically, using the MitoCheck Complex Activity Assay Kit (Cayman Chemical Company, Ann Arbor, MI, USA), extent of relative activity of each of Complex I, Complex II, Complex II/III, Complex IV and Complex V constituting the mitochondrial respiratory chain that was reduced by neopetasin treatment was examined. Furthermore, as a negative control section, DMSO was administered to perform the same experiment. In addition, as a positive control section, rotenone (Complex I), TTFA (Complex II), antimycin A (Complexes II/III), KCN (Complex IV), and oligomycin (Complex V) were each administered, and the same experiment was conducted.

The results are shown in Fig. 10. As illustrated in Fig. 10, neopetasin selectively reduced only the relative activity of the mitochondrial respiratory chain Complex I. From this, it was shown that neopetasin acts as a mitochondrial toxin by inhibiting the mitochondrial respiratory chain Complex I.

### (Test Example 10)

Western blotting was used to examine changes in expression of various cancer-related proteins in neopetasin-treated cells.

KMeC cells (canine malignant melanoma cells) were obtained from the Veterinary Surgery Laboratory, Graduate School of Agricultural and Life Sciences, the University of Tokyo. KMeC cells were cultured in an incubator (air/CO₂ = 95/5 (v/v)) at 37°C using RPMI1640 medium.

To start with, each cell was seeded at 0.5×10⁵ cells/mL in a six-well plate, and after 24 hours of seeding, neopetasin (final concentration: 3 µM) was suspended in a medium and incubated for 56 hours. B16F10 cells thus treated with neopetasin (3 µM) for 56 hours were recovered with a cell scraper. Furthermore, DMSO was administered to the negative control section, and the same experiment was performed.

Next, the cells were homogenized in ice-cold lysis buffer (10 mM Tris-HCl (pH 7.4), 1% (w/v) NP-40, 0.1% (w/v) deoxycholic acid, 0.1% (w/v) SDS, 150 mM NaCl, 1 mM EDTA, and 1% (w/v) protease inhibitor cocktail (Sigma-Aldrich) and allowed to stand on ice for 20 minutes. The homogenate was centrifuged at 13,000 rpm for 20 minutes (4°C), and then the supernatant was collected as a sample for Western blotting. The protein content in the sample was measured using a DC protein assay kit (manufactured by Bio-Rad Laboratories, Inc.). A sample (protein amount of 5 µg) was separated by SDS-PAGE using 10.0 or 12.5% (w/v) polyacrylamide gel, and transferred to a PVDF membrane (PerkinElmer Life Science Co., Ltd). Nonspecific binding was blocked by incubation in 5% (w/v) degreased milk (prepared with PBS containing 0.1% (w/v) Tween (registered trademark) 20 (PBS-T)) for one hour. Afterwards, the membranes were incubated overnight at 4°C with primary antibodies appropriately diluted in TBS-T containing 2% (w/v) bovine serum albumin and 0.01% (w/v) sodium azide. Next, the membranes were washed three times with PBS-T and further incubated with a secondary antibody (HRP-conjugated anti-mouse or anti-rabbit IgG antibody, Cell Signaling Technology) at room temperature. Then, the membranes were washed three times with PBS-T. Immunoblots were visualized using Amasham ECL plus Western blotting detection reagent (GE Healthcare) . β-actin was used as an internal standard by re-incubating the same membranes using an anti-β-actin antibody (Sigma-Aldrich Co. LLC) .

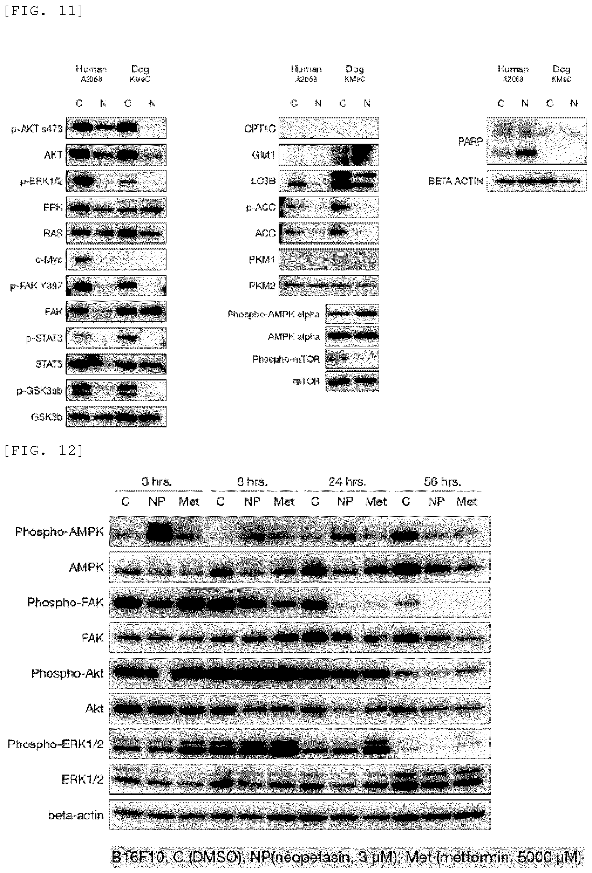

The results are shown in Fig. 11 (in Fig. 11, "C" represents a negative control section, and "N" represents the results of neopetasin-treated cells). As illustrated in Fig. 11, neopetasin was shown to inhibit a wide range of cancer-related molecules in both A2058 cells and KMeC cells. In other words, according to the results shown in Fig. 11, it can be seen that in neopetasin-treated cells, reduction of phosphorylation (activated type) of molecular groups (Akt, ERK1/2, FAK, and STAT3) related to cancer proliferation and metastasis, changes in molecular groups (AMPK, mTOR, ACC, and GSK3b) that regulates the cancer metabolism, and apoptosis induction (degradation of PARP), etc. occurred.

### (Test Example 11)

Western blotting was used to examine temporal changes in the expression of various cancer metabolism/proliferation/metastasis related proteins (AMPK, FAK, Akt, ERK1/2) in B16F10 cells treated with neopetasin or metformin.

Specifically, for B16F10 cells treated with neopetasin (3 µM) or metformin (5000 µM) through the same method as in Test Example 1, the cells were recovered at each of 3 hours, 8 hours, 24 hours, and 56 hours after the treatment with these compounds, and the contents of various proteins were measured by Western blotting through the same method as in Test Example 10. Furthermore, DMSO was administered to the negative control section, and the same experiment was performed.

The results are shown in Fig. 12 (in Fig. 12, "C" represents a negative control section, "NP" represents results of neopetasin-treated cells, and "Met" represents results of metformin-treated cells). According to the results shown in Fig. 12, it can be seen that AMPK, which is a master regulator regulating the cancer metabolism, changes at the earliest stage (3 hours) after the neopetasin treatment, FAK, which is associated with metastatic adhesion, is then inactivated, and Akt and ERK, which are central molecules that ultimately control metastasis, proliferation, and death of cancer cells, are inactivated. From this, it was suggested that neopetasin exerts inhibitory effects on proliferation and metastasis inhibitory function of tumor cells by regulating the cancer metabolism.

### (Test Example 12)

In general, the metastasis process follows the course of primary tumor formation, invasion of blood vessels and lymphatic vessels, and fixation and proliferation to lung. Here, the model prepared in Test Example 2 verifies only kinetics of tumor cells after entry into the blood vessel, and the metastasis phenomenon occurring in the actual clinical example also includes other processes in addition to invasion of blood vessels and lymphatic vessels from the primary tumor. Thereupon, in this test example, a subcutaneous tumor (a primary tumor) was generated in a mouse, and a model in which the tumor cells metastasized to lung following all metastasis processes (a natural metastasis model) was prepared. In addition, by administering neopetasin to such natural metastasis model, whether the same metastasis inhibitory function as described above was exhibited was examined.

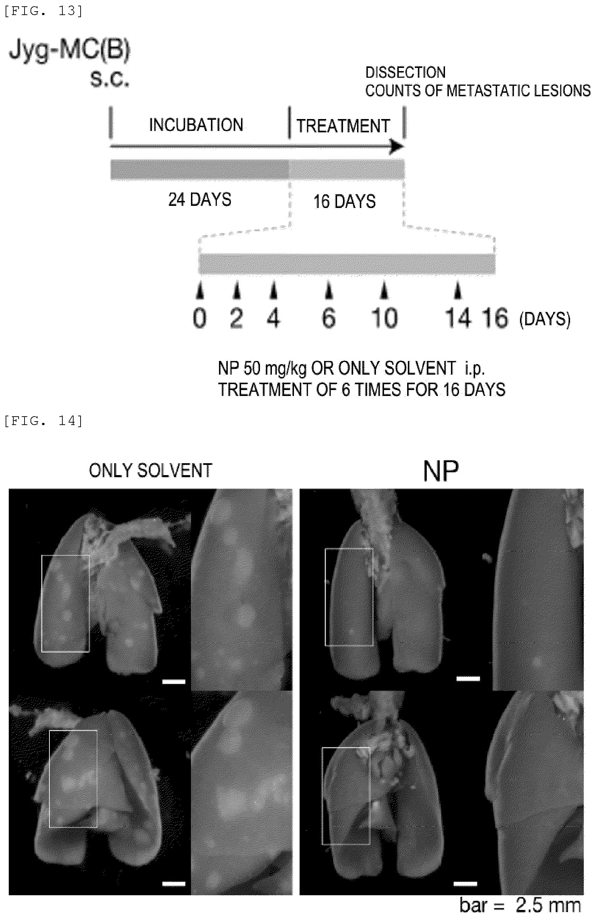

Specifically, to start with, in order to prepare a natural metastasis model, a nude mouse (BALBc nu/nu, female, 4 weeks old, purchased from Japan SLC) was subcutaneously inoculated with mouse-derived metastatic breast cancer cells Jyg-MC(B). After 24 days of incubation, neopetasin (NP) was administered intraperitoneally at a dose of 50 mg/kg and treated a total of 6 times for 16 days (inoculation at 0, 2, 4, 6, 10, 14 days, respectively) . Note that treatment procedure at this time is illustrated in Fig. 13.

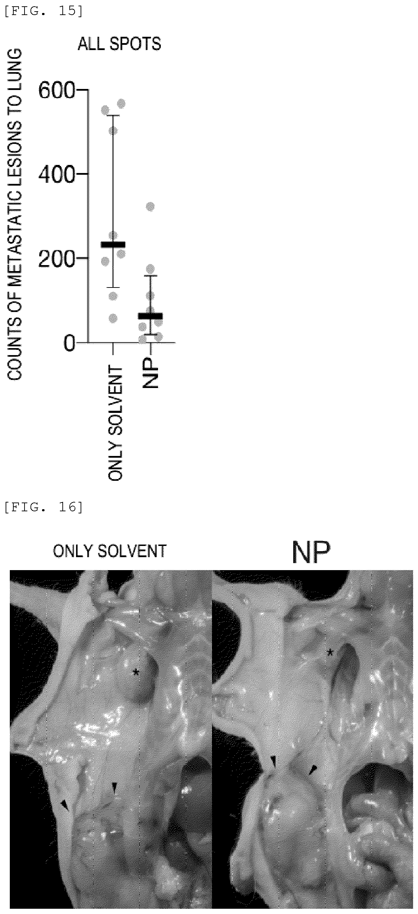

As a result, it was confirmed that neopetasin exhibited a metastasis inhibitory function also in the natural metastasis model. Specifically, in neopetasin-treated group, a significant decrease in lung metastasis lesions was observed as compared with the control groups. Furthermore, Fig. 14 shows a macroscopic photograph of the lung extracted from each group of mice, and Fig. 15 shows a graph comparing the number of metastasis lesions in surface layer of each lung.

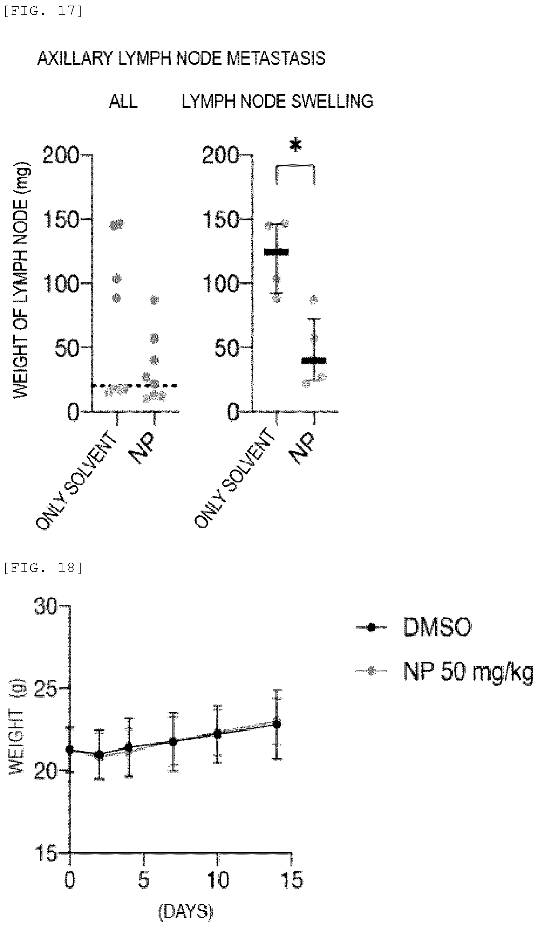

Furthermore, the weight of the axillary lymph node was measured in order to examine the influence on the lymphatic metastasis. As a result, it was found that neopetasin (NP) treatment also exhibits an inhibitory effect on lymph node metastasis. Moreover, Fig. 16 shows a macroscopic photograph of the axillary lymph node extracted from each group of mice. In Fig. 16, * indicates the axillary lymph node, and the arrowhead indicates the primary tumor. In addition, results of measuring the weight of the lymph node for each of all the axillary lymph nodes or the swollen axillary lymph nodes in each group are shown in Fig. 17.

In the model produced in this test example, since the lymph node metastasis is the phenomenon occurred at a frequency of about half, the weight was compared excluding the example in which no lymph node swelling was observed. As a result, it was found that degree and weight of the swelling decreased in the NP-treated group. In addition to the above, under the present administration conditions, side effects such as weight loss due to NP treatment and abnormalities and variations in blood test were not observed (Fig. 18).

From the above, it was demonstrated that neopetasin exhibits a strong anti-metastatic effect even in a natural metastasis model that includes a more complicated process. Since the natural metastasis model has a metastasis mechanism equivalent to the phenomenon occurring in actual clinical examples, it is expected that neopetasin can exhibit a potent anti-metastatic effect also in the actual clinical examples based on the results of this test example.

### (Test Example 13)

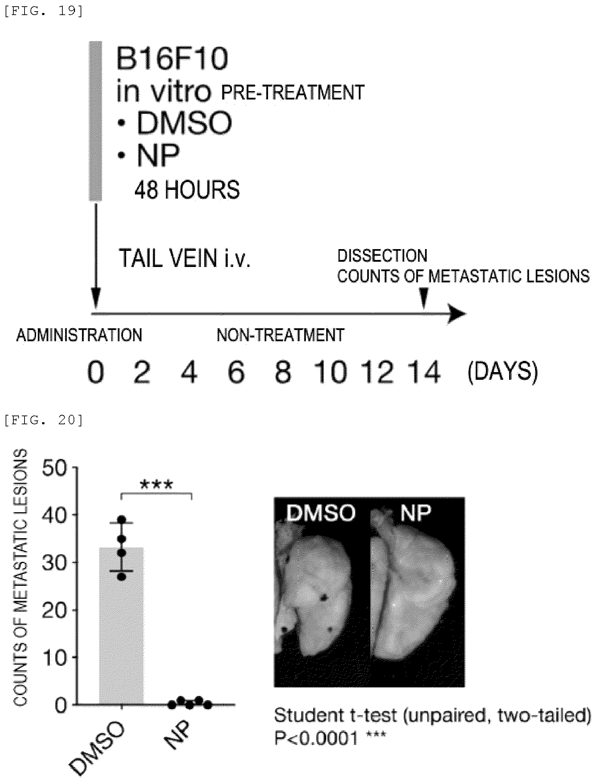

For the purpose of confirming that the proposition "when proliferation of tumor cells is suppressed by anticancer agent, metastasis of tumor cells is also suppressed" is incorrect, the following experiment was performed. In this test example, neopetasin (NP) was previously reacted with B16F10 cells that are derived from the mouse melanoma at a concentration of 3 µM in vitro. Thereafter, the uniform numbers of living cells were injected into the tail vein of the mice, and thereafter, no treatment was performed, and the number of metastasis lesions formed in the lung after a two-week non-treatment period was counted. The protocol of the present test example is shown in Fig. 19, and the graph showing the results of the present test example and the observation photograph of the extracted lung are shown in Fig. 20. According to the graph and observation photographs shown in Fig. 20, it is found that the formation of metastasis lesions was not observed even after the non-treatment period in neopetasin (NP)-administered group, whereas many metastasis lesions were formed in the control (DMSO-administered group). Here, if neopetasin (NP) were reducing the number of lung metastasis lesions as part of the proliferation inhibitory effect on tumor cells (in other words, lung metastasis is suppressed as part of the proliferation inhibitory effect), equivalent formation of metastasis lesions should be observed in both neopetasin (NP)-administered group and the control (DMSO-administered group) during the non-treatment period in which the proliferation inhibitory effect is not exerted. However, in reality, as described above, formation of a new metastatic lesion has not been confirmed even after the non-treatment period in neopetasin (NP)-administered group. Therefore, it is found that neopetasin (NP) exerts a metastasis inhibitory effect on tumor cells as a separate action independent of the proliferation inhibitory effect on tumor cells. It can be said that this fact indicates that the proposition "when proliferation of tumor cells is suppressed by anticancer agents, metastasis of tumor cells is also suppressed" is not always correct. In addition, the results of this Test Example and the results of Fig. 3 of the literature described above (Paez-Ribes, M. et al. Antiangiogenic Therapy Elicits Malignant Progression of Tumors to Increased Local Invasion and Distant Metastasis. Cancer Cell 15, (2008).) also strongly suggest that the proliferation of tumor cells and the metastasis of tumor cells are performed via different mechanisms.

### (Test Example 14)

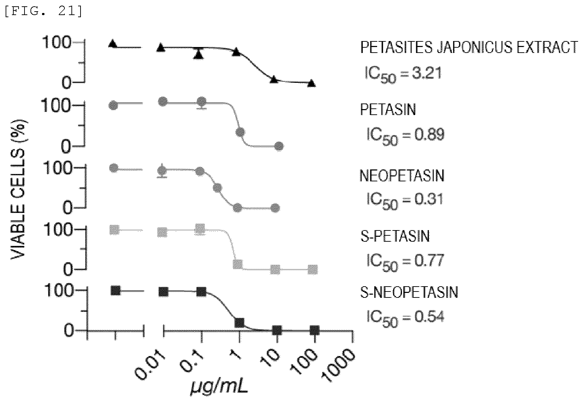

In this test example, IC₅₀ values in the cell proliferation inhibition assay for petasin, neopetasin, S-petasin and S-neopetasin were measured in addition to the Petasites japonicus extract (whole extract). The results are shown in Fig. 21. From the results shown in Fig. 21, it is found that neopetasin exhibits superior cell proliferation inhibition activity to the other three compounds. Moreover, also as shown in Fig. 1C, neopetasin shows no cytotoxicity against ASF4-1 cells, which are normal human skin derived fibroblasts. In this way, it can be said that neopetasin, which exhibits metastasis inhibitory activity on tumor cells without exhibiting cytotoxicity to normal cells, is extremely superior, especially, as a metastasis inhibitor of tumor cells among the compounds according to the present invention.

### (Summary)

To summarize the results shown in each of the Test Examples described above, to start with, a tumor cell lung metastasis test was performed using B16F10 cells, which are highly metastatic malignant melanoma cell lines in mice, and it was found that neopetasin administration inhibited lung metastasis of B16F10 tumor cells. Furthermore, it was confirmed that this metastasis inhibitory function was similarly expressed in a natural metastasis mouse model. Moreover, in agreement with the specificity of the antitumor effect in vitro, neopetasin administration showed little toxicity to the liver, kidney, spleen, and the like. As a result of searching for an effect expression mechanism, it was found that neopetasin disrupts tumor metabolism, and particularly inhibits aspartic acid synthesis to 1/10 or less. Here, aspartic acid is an amino acid synthesized in the mitochondria and it is essential for synthesis of nucleic acids or proteins. As described above, neopetasin is considered to primarily exert an action by disrupting amino acid metabolism, and eventually strongly suppresses energy metabolism and also synthesis of nucleic acids from the glycolytic pathway via pentose phosphate cycle. In addition, by significantly decreasing expression of FAK, cell movement and morphological change are impaired. From the above, it is considered that neopetasin inhibits metastatic potential of tumor cells by potently inhibiting the aspartic acid synthesis, which is a nutrient required for proliferation and adaptation at metastasis sites of tumor cells.

Furthermore, it has also been found that the inhibitory action on the aspartic acid synthesis by neopetasin as described above is due to direct and potent inhibition of the mitochondrial respiratory chain complex I. Moreover, it was also found that the activity of such neopetasin was 1000 to 10000 times or more higher than metformin or phenformin.

This application is based on Japanese Patent Application No. 2020-022827 filed on February 13, 2020, the contents of which are incorporated herein by reference in its entirety.

## Claims

1. An aspartic acid synthesis inhibitor in tumor cells, comprising, as an effective component, a compound represented by the following Chemical Formula 1A or the following Chemical Formula 1B: 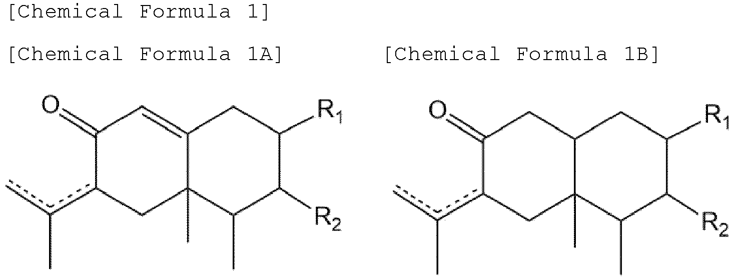 wherein R₁ and R₂ are each independently a hydrogen atom, a hydroxyl group or an optionally substituted hydrocarbon group; and one of two adjacent chemical bonds indicated by dotted lines is a single bond, and the other is a double bond.

2. The inhibitor according to claim 1, wherein the compound is petasin or neopetasin.

3. A spheroid formation inhibitor of tumor cells, comprising the compound according to claim 1 as an effective component.

4. The spheroid formation inhibitor according to claim 3, wherein the compound is petasin or neopetasin.

5. A metastasis inhibitor of tumor cells, comprising the inhibitor according to any one of claims 1 to 4.

6. The metastasis inhibitor according to claim 5, wherein the tumor cells are cells of malignant melanoma.

7. The metastasis inhibitor according to claim 5 or 6, wherein the metastasis inhibitor suppresses metastasis of the tumor cells to lung.

8. An action enhancer of glycolysis inhibitors, comprising the compound according to claim 1 as an effective component.

9. The action enhancer according to claim 8, wherein the compound is petasin or neopetasin.

10. The action enhancer according to claim 8 or 9, wherein the glycolysis inhibitor is 2-deoxy-D-glucose.

11. A pharmaceutical composition for suppression and/or prevention of metastasis of a tumor, comprising the action enhancer according to any one of claims 8 to 10.

12. The pharmaceutical composition according to claim 11, wherein the pharmaceutical composition is administered in combination with a glycolysis inhibitor.

13. The pharmaceutical composition according to claim 12, wherein the glycolysis inhibitor is 2-deoxy-D-glucose.

14. The pharmaceutical composition according to any one of claims 11 to 13, wherein the pharmaceutical composition is orally administered to a human or a pet animal for use.

15. A food or drink composition that suppresses metastasis of tumor cells, comprising the compound according to claim 1 as an effective component.

16. The food or drink composition according to claim 15, wherein the compound is petasin or neopetasin.

17. The food or drink composition according to claim 16, wherein the composition is a human health food or a pet food composition.
